# EUROPEAN PATENT SPECIFICATION

(11) **EP 1 214 397 B1**
(45) Date of publication and mention of the grant of the patent: **17.03.2004**
(21) Application number: 00964630.8
(22) Date of filing: 21.09.2000
(51) Int. Cl.: C12N 5/06, A61K 31/40, A61K 38/04, C12Q 1/02, A61P 35/00

(54) **METHOD FOR THE SELECTIVE PROTECTION OF PROLIFERATING NORMAL CELLS AND THE SELECTIVE ERADICATION OF TUMOR CELLS HAVING AN INACTIVE P53 PATHWAY**
METHODE FÜR DIE SELEKTIVE PROTEKTION DER PROLIFERATION DER NORMALEN ZELLEN UND DER SELEKTIVEN BESEITIGUNG DER TUMORZELLEN, DIE EINE INAKTIVIERTE P53 PATHWAY HABEN
METHODE DE PROTECTION SELECTIVE DE CELLULES NORMALES PROLIFERANTES ET D'ERADICATION SELECTIVE DE CELLULES TUMORALES AYANT UNE VOIE P53 INACTIVE

(30) Priority: 21.09.1999 IT RM990580
(43) Date of publication of application: 19.06.2002
(73) Proprietor: UNIVERSITA' DEGLI STUDI DI ROMA "LA SAPIENZA", 00185 Roma (IT)
(72) Inventor: TATO', Franco, I-00189 Roma (IT); ZANETTI, Antonio, I-00152 Roma (IT); GROSSI, Milena, I-00151 Roma (IT)
(74) Representative: Germinario, Claudio
(86) International application number: PCT/IT2000/000373
(87) International publication number: WO 2001/021765

(56) References cited:
- EP-A- 0 297 946
- WO-A-97/10242
- RICHMAN C M ET AL: "INTERFERON PROTECTS NORMAL HUMAN GRANULOCYTE-MACROPHAGE COLONY-FORMING CELLS FROM ARA-C CYTOTOXICITY" JOURNAL OF BIOLOGICAL RESPONSE MODIFIERS, vol. 9, no. 6, 1990, pages 570-575, XP002165025 ISSN: 0732-6580
- SASSE FLORENZ ET AL: "The chondramides: Cytostatic agents from myxobacteria acting on the actin cytoskeleton." JOURNAL OF THE NATIONAL CANCER INSTITUTE (BETHESDA), vol. 90, no. 20, 21 October 1998 (1998-10-21), pages 1559-1563, XP000990945 ISSN: 0027-8874
- BOKEMEYER C ET AL: "SILIBININ PROTECTS AGAINST CISPLATIN-INDUCED NEPHROTOXICITY WITHOUT COMPROMISING CISPLATIN OR IFOSFAMIDE ANTI-TUMOUR ACTIVITY" BRITISH JOURNAL OF CANCER,GB,LONDON, vol. 74, 1996, pages 2036-2041, XP000874389 ISSN: 0007-0920
- OTSUKA H ET AL: "VARIANT LINES OF MOUSE KIDNEY CELLS TRANSFORMED BY AN SV-40-TSA MUTANT WITH GROWTH PROPERTIES OF WILD TYPE TRANSFORMED CELLS AT NONPERMISSIVE TEMPERATURE" JOURNAL OF GENERAL VIROLOGY, vol. 42, no. 2, 1979, pages 373-386, XP000990951 EN ISSN: 0022-1317
- RUBTSOVA S N ET AL: "Disruption of actin microfilaments by cytochalasin D leads to activation of p53." FEBS LETTERS, vol. 430, no. 3, 3 July 1998 (1998-07-03), pages 353-357, XP002164902 ISSN: 0014-5793
- WANG E A ET AL: "BONE MORPHOGENETIC PROTEIN-2 CAUSES COMMITMENT AND DIFFERENTIATION IN C3H10T1/2 AND 3T3 CELLS" GROWTH FACTORS,HARWOOD ACADEMIC PUBLISHERS GMBH,XX, vol. 9, no. 1, 1993, pages 57-71, XP002920878 ISSN: 0897-7194

## Description

### Field Of The Invention

The present invention relates to an in vitro method and a use that allows to protect normal cells while eradicating tumor cells, in particular the ones having a low proliferation rate, from a context wherein both tumor and normal cells are present and proliferate.

### Background of the invention

The main purpose of any cancer treatment is the complete and at the same time selective eradication of tumor cells from patient tissues.

Completeness of tumor cells eradication is in fact essential for preventing recidivating forms, selectivity instead for keeping the patient safe from side effects.

Both features are therefore essential for a successful cancer treatment, even if the full attainment of both at the same time is extremely difficult, in view of the opposite technical effects underlying each of them ("killing cells" vs. "saving cells").

In the *in vitro* system applications related thereto (i.e. directed to search new therapeutic approaches or new therapeutic compounds) instead, even a partially selective and/or a partially complete eradication of the tumor cells could instead be also advantageous if controllable, as controllability ensures a stronger reliability of the data.

In current therapeutical approaches the complete eradication of tumor cells *in vivo* is attempted by repeated administration of chemotherapeutic agents, in addition to other ablatory intervention like radiotherapy and surgery if any.

As such, chemotherapeutic agents have generally the capability of effectively killing proliferating cells in a preferential way; a therapeutic approach making use of said agents allows some selective eradication of tumor cells with respect to normal cells, that are usually in a non-proliferating phase.

There are however some normal cells which physiologically proliferate and are therefore with respect to chemotherapeutic agent action, totally equivalent to tumor cells.

Such an "aspecificity" characterizing chemotherapeutic agent action on proliferating cells clinically results in a marked and general toxicity on proliferating tissues like bone marrow, gastrointestinal mucosae and skin.

Hence the treatment interruption is often the only clinical solution to side effects deriving from such a marked toxicity, and consequently, regardless of the remarkable therapeutic potential of the chemotherapeutic drugs, the complete eradication of the tumor cells in most patients is hard to attain.

This is in fact for instance the standard practice, in case of toxicity toward gastrointestinal mucosae, whereas, in case of toxicity toward the hematopoietic precursors, other approaches are also practiced which however have been proved to be not decisive in a significant percentage of cases. (1)

However, in view of their highly powerful eradicating activity, most chemotherapeutic compounds, even in presence of side effects, can be effectively used for treating forms with high proliferation potential, even if with heavy costs in term of patient suffering.

No use is instead practicable in forms having low proliferation potential, like pancreatic cancer and some lymphomas. Being characterized by a proliferation rate nearly identical to that of replicating normal tissues, such tumor forms in fact would require treatment times and chemotherapeutic dosages that would aspecifically kill also the totality of the proliferating normal cells.

Hence, there is an urgent need for alternative therapeutical approaches, capable of protecting proliferating normal cells from the toxic action of the chemotherapeutic drugs.

Although some alternative approaches in this connection have been developed in art, an efficient protection of the normal cells corresponding to a complete and selective eradication of the tumor cells, has not yet been obtained.

The main difficulty in reaching such a result lies in the too similar characteristics shared by proliferating normal and tumor cells, so that, with respect to the action of the chemotherapy drug, they are nearly undistinguishable.

Accordingly, one of the possible approaches to solve the problem of chemotherapy-associated side effects is to define and find drugs that can help to preserve proliferating normal cells from the adverse effects of already known chemotherapeutic drugs.

Some approaches have been therefore studied *in vitro* in order to find out compounds capable of establishing a condition in which, with respect to chemotherapeutic intervention, normal cells exhibit a remarkably reduced or an absent sensitivity, whereas the sensitivity of the tumor cells is unaltered (herein referred also as "differential status"). However, no significant data in an applicative and therapeutic perspective have yet been obtained.

Accordingly, there is still the requirement for experimental settings and cell models apt to define a novel rationale adequate to make feasible the possibility of defining conditions and mechanisms capable of protecting proliferating normal cells from the toxic effects of chemotherapeutic agents.

The best system to this purpose in fact would obviously be the one constituted by proliferating normal human cells that are subject *in vivo* to chemotherapy-associated side effects, i.e. proliferating stem cells of physiologically self-renewing tissues. Such cells, however, modify their proliferative state when transferred *in vitro,* and are therefore not suitable to produce data relevant to *in vivo* situations.

In the absence of a such a kind of cell system, at least partially reproducing an *in vivo* situation, no experiments directed to select compounds and approaches in an applicative perspective, would have been meaningful.

### Summary Of The Invention

A first object of the present invention is a method for protecting proliferating normal cells in an *in vitro* or *ex vivo* culture comprising said proliferating normal cells and tumor cells having an inactive p53 pathway, from the eradicative action of a chemotherapeutic compound (herein also referred as class B compound or drug) having the capability of
- exerting a cytotoxic action toward actively proliferating cells and
- not affecting survival and proliferative potential of interphase cells,
comprising
a combined treatment including administering to said culture a protective compound (herein also referred as class A compound or drug) having the capability of
- reversibly inhibiting cytodieresis of normal cells and
- non-inhibiting the biological action of said class B, chemotherapeutic compound,
in combination with said chemotherapeutic compound,
the administration of said protective compound resulting in the protection of at least part of said proliferating normal cells.

The method of the present invention is based on the surprising observations that the binucleation/multinucleation state induced by class A compounds in normal/tumor cells respectively, is a "differential status" as herein defined with respect to the eradicative action of class B compounds, and that such a "differential status" is strictly dependent on the functioning of the p53 pathway in normal cells and the lack of a functional p53 pathway in most tumor cells.

In the method of the present invention said "differential status" is therefore induced by a class A compound in proliferating normal cells having an active p53 pathway, but not,in tumor cells having an inactive p53 pathway, thus protecting said proliferating normal cells from the toxic effects of class B compounds, which are a specific subset of chemotherapeutic drugs targeting replicating cells.

Such a protection entails the reversible inhibition of cell division that leads to inhibition of further DNA replication in normal cells, but not in tumor cells displaying an inactive p53 pathway. Consequently, a high degree of selectivity toward tumor cells is conferred to otherwise unselective drugs, so that it becomes feasible to reach a complete eradication of tumor cells, particularly those with a low proliferation rate, while sparing normal cells, from a context wherein both normal and tumor cells are present and proliferate.

The reversibility of the class A compound action, upon drug removal, allows subsequent recovery of physiological proliferative ability of said normal cells.

Accordingly, in outcome to the method of present invention a complete protection of many normal cells which have been arrested in the intermitotic phase by class A compound action, from the eradicative action of the class B compound, and a complete eradication of the tumor cells having an inactive p53 pathway, is obtainable.

Further, according to the present invention both protection of normal cells and eradication of tumor cells are controllable functions of certain parameters (CT: time of the combined treatment; CACT: concentration of class A compound in the combined treatment; and CBCT: concentration of class B compound in the combined treatment) as evidenced below.

Accordingly, protection of normal cells and eradication of tumor cells can be controlled up to the complete and selective eradication of all the tumor cells from a mixed culture and a significant and selective protection of normal cells in the culture.

In a preferred embodiment before the combined treatment a pretreatment is carried out comprising administering to said culture the protective compound as defined above, the administration of said protective compound resulting in the arrest at interphase of at lest part of said normal cells.

In this case, as the arrest of normal cell to be protected is carried out before administration of class B compound, the protection obtainable thereby is more controllable up to be complete for many normal cells capable of proliferating in the culture.

In this treatment analogously to combined treatment protection of normal cells is a function of certain parameters (PT: pretreatment time; and CAP: concentration of the class A compound used in pretreatment) as evidenced below.

In any case for obtaining the most complete protection of normal cells it has to be taken into account that due to the reversibility of the action of the class A compounds on the normal cells, the concomitant interruption of the treatment with both compounds can determine the death of a small, but sizeable percentage of normal cells.

In fact, this effect is to be ascribed to the action of the residual class B compound onto the normal cells, that, in the absence of class A compound, promptly restart to proliferate.

In this connection, although the administration of class A and class B compound can be interrupted contemporaneously or at different time according to the experimental purpose, in a preferred embodiment after the combined treatment a post treatment is carried out, including a washing step wherein
- after interrupting the administration of said class B compound, said class B compound is washed off the culture, the administration of said class A compound being maintained.

After this washing step the administration of the class A compound can be interrupted according to the experimental purpose.

In the method wherein the washing step is carried out any possible residual cytotoxic effect of class B compound on normal cells due to contemporaneous interruption of the administration of class A and class B compounds, can be avoided.

As a consequence the further advantage of the method of present invention in the embodiment including post-treatment is that, in case the pretreatment results in the arrest of all the normal cell having capability to proliferate, the fully selective and complete eradication of any tumor cell type having an inactive p53 pathway, and the corresponding full protection of any normal cells in culture is thereby obtainable.

A class A compound (capable of inducing such a "differential status" between normal cells and tumor cells having an inactive p53 pathway) according to the present invention is any compound having the following features:
- capability of inhibiting cytodieresis in normal cells
- reversibility of said inhibition of cytodieresis in normal cells and
- capability of non-inhibiting the biological action of a class B compound.

The capability of inhibiting cytodieresis according to the present invention is the ability to inhibit daughter cell division, and hence the subsequent phases of cell cycle, in proliferating normal cells having a functional p53 pathway, by interfering directly or indirectly, with the remodeling of the actomyosin cytoskeleton.

It generally results in cell cycle arrest at the binucleate stage in at least 40% of class A compounds treated proliferating normal cells, using the minimal drug concentration (i.e. sufficient to reversible induce its effects, on target cells, without causing significant damage, experimentally defined by dose/response curves), giving the above result without significant toxicity and an incubation time generally ranging from 12 to 48 hours depending on cell type.

Reversibility after drug removal of said inhibiting capability is associated to the recovery of a proliferative potential similar to that displayed by normal cells before class A drug treatment.

Said reversibility of the class A compound effects, after drug removal, generally includes:
- a transition from binucleate to mononucleate cells of at least 60% of cells;
- a recovered competence for DNA synthesis, in at least 70% of cells within 48 hours after drug removal;
- most of all, a recovery of at least 30% of the clonogenic potential as compared to untreated control cells.

Concerning instead the capability of non-inhibiting the biological action of a class B compound, it generally results in the reduction of a class B compound uptake by the tumor cells having an inactive p53 pathway of a percentage lower or equal to 50%.

Specific preferred class A compounds, are compounds belonging to families of cytochalasins (2, 3, 4, 5), with the exclusion of cytochalasin B (6, 7, 8), jasplakinolides (9), chondramides (10), isoindolinones (11), and the latrunculines(12). In particular cytochalasin D, dihydrocytochalasin B jasplakinolide, chondramide B and latrunculin B.

A class B compound (eradicative and/or chemotherapeutic agent) according to the present invention is instead any compound having the capability of displaying cytotoxicity toward actively proliferating cells, without affecting the proliferating potential and the viability of interphase cells.

Cytotoxicity toward low density (10² - 10³ cells/cm²)actively proliferating cells is a toxicity generally resulting in a reduction of at least 90% of the number of surviving proliferating cells (as compared to untreated control cells), treated with the appropriate (i.e. capable of causing an irreversible loss of viability in at least 90% of the cells, as deduced from dose/response experiments) drug concentration for a time ranging from 24 to 96 hours.

As concerns the capability of not affecting proliferative potential of interphase cells, it is associated to the capability of class B compound of being not cytotoxic towards normal cells arrested in the intermitotic phase, as a consequence to the class A action. It generally results after treatment interruption in:
- maintenance of a constant interphase cell number upon 24-48 hours treatment, corresponding to at least 80% of interphase cell number at the beginning of the experiment as compared to untreated control cells;
- induction of DNA synthesis by serum stimulation preferably in at least 40% of interphase cells after 24-48 hours drug treatment as compared to untreated control cells.

Specific preferred class B compounds, are compounds belonging to families of folate inhibitors, nucleoside analogues, nucleotide synthesis inhibitors, vinca alkaloids, taxanes, colchicine derivatives, podophillotoxin derivatives, and topoisomerase inhibitors and in particular are trifluorothymidine (2'-deoxy-5-trifluoromethyluridine), cytarabine, 6-thioguanine, 6-mercaptoputrine, gemcytabine, fludarabine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, 9-amino-20(S)-camptothecine. All these compounds target different phases of cell cycle and share a high degree of toxicity for highly proliferating cells (1)

With regard to the treatments of the method of the present invention such class A and class B compounds are used in combination, as reported above they are function of certain parameters.

With regard to the pretreatment, the relevant parameters are pretreatment time (PT) and concentration of the class A compound used in the pretreatment step (CAP). The PT, is the time necessary for inducing binucleation in normal cells to protect them, according to the experimental purpose. PT length is therefore function of the cell cycle time of normal cells that are to be protected. *In vitro* such a time may differ among various cell types as different cell types have different cell cycle times (e.g., under standard condition a primary fibroblast has a cell cycle of about 24-48h, the C3H10T1/2 of 20 hours). However, a person skilled in the art could predetermine it for each cell type on the basis of the state of the art.

In *ex vivo* treatment instead it can be univocally predetermined for all cell types, since the cell cycle time *ex vivo* (like *in vivo)* is highly standardized.

In the preferred embodiment, wherein the totality of the normal cells in culture are to be protected, the PT is greater than or equal to cell cycle duration of said proliferating normal cells both *in vitro* or *ex vivo* (in both instances it is the time required by most of the physiologically proliferating normal cells in the culture for arresting their growth). According to some experimental data, under standard conditions this time ranges between 12 and 96 hours, depending on the cell types.

However, according to what set forth above, PT may be lengthened or shortened, depending on the different proportion of proliferating normal cells to be protected in the different applications.

As to the CAP parameter, the corresponding value is a class A compound concentration causing the arrest of at least the 40% of normal cells in the intermitotic stage; such concentration is experimental deduced by dose/response experiments that define the appropriate value range for different cell types and compounds.

The relevant values vary as a function of the class A compound and the cell type used, but according to what set forth above it can be determined for each single class compound by those skilled in the art on the basis of the state of the art knowledge and on the teaching of the present invention.

For the above class A compounds, under standard conditions this value ranges within 1 nM to 40 µM concentrations.. Under standard conditions for instance, the CAP value for cytochalasin D is preferably comprised within the range 20 nM-2 µM, and in a preferred embodiment it is of 400 nM.; for the dihydrocytochalasin B, it is comprised in the range 600 nM - 8µM, and in a preferred embodiment it is of 2µM; for the jasplakinolide, it is comprised in the range 50 nM - 3µM and in a preferred embodiment it is of 700nM; for the latrunculin B it is comprised in the range 500nM - 2 µM and in a preferred embodiment it is of 650nM; for the chondramide B, it is comprised in the range 10 - 800 nM and in a preferred embodiment it is of 100nM. Said preferred concentrations are the CAP value corresponding to the minimum concentration under standard conditions required for reaching the arrest in intermitotic phase of the normal cells to be protected (minimum functional concentration), but could also be higher.

Like PT also CAP can in fact however be varied in function of the desired protection to be attained. Generally the combination CAP-PT can be varied by a person skilled in the art on the basis of the above information and of the state of the art in function of the desired protective effect.

With regard to the combined treatment, the relevant parameters are CT (time of the combined treatment), CACT (concentration of class A compound in the combined treatment) and CBCT (concentration of class B compound in the combined treatment).

With regard to TC, this time is functional to the tumor cell eradicative effects desired. Since class B compounds kill replicating cells, the optimal CT can be predicted to be equivalent to the average cell cycle time of the tumor cells to be eradicated, following the rule the longer the treatment the best the results.

Accordingly CT is a function of the cell cycle of the tumor cells targeted by the class B compound, in that it can be equal to, lower then or greater than tumor cell cycle time, according to the different eradication protocols that can be planned.

In the preferred embodiment wherein all the tumor cells having an inactive p53 pathway are to be eradicated, such a time is generally greater than or equal to cell cycle duration of tumor cells having an inactive p53 pathway to be eradicated. Preferably it is the minimum time needed by the tumor cells having inactive p53 pathway to complete at least one cell cycle, which is the time within which, in absence of administration of a class A compound, all the tumor cells inside the culture are eradicated by the class B compound.

CT however can be also increased up to two or more cell cycle equivalents, in order to eradicate even those tumor cells that might have survived after the first cell cycle. However, CT equivalent to two tumor cell cycles constitutes no top limit and it can be further increased as required.

Such a time can also be decreased from one cell cycle time, in cases where a more gradual eradicative effect is required.

In any case the cell cycle time of tumor cells varies *in vitro* from cell type to cell type (as observed above for the normal cell cycle time). CT varies according to the cell type to be eradicated.

Since cell cycle time can be determined for any cell type by a person skilled in the art, on the basis of the state of the art, also CT can be determined accordingly by a person skilled in the art, always in function of the eradicative desired effect.

Concerning the concentration of the class A compound to be administrated in the combined treatment (CACT), it is the concentration necessary for obtaining the arrest in the intermitotic phase of at least 40% of the normal cells that are to be protected. Thus, in a manner in all analogous to the CAP, CACT varies in function of the class A compound and of the cell type used.

In view of what set forth above regarding the class A compound action the corresponding value can be determined for each individual class compound by a person skilled in the art, on the basis of the knowledge of the state of the art and of the present application.

CACT generally corresponds to the CAP, and therefore under standard conditions for the above mentioned compounds it can be comprised within the concentration range indicated for the pretreatment. Although, the CACT is usually equal to CAP, it could even be arbitrarily increased or lowered within or outside the mentioned range. In the preferred embodiment, the class A compounds are uninterruptedly administered during both phases.

Concerning the concentration of class B compound (CBCT), it is comprised in a range that should be calculated as a function of the CT, the target tumor cell type and the half-life in culture of the administered class B compound. In particular CBCT is equal to the drug concentration capable of killing at least 50% of the target tumor within the length of one cell cycle time, unless a more gradual rate of eradication is required. Usually, under standard culture conditions for the above class B compounds it is greater than or equal to 100nM.

In particular, for the trifluorothymidine this concentration is greater than or equal to 40 µM, in particular comprised in the range 10µM - 800 µM, preferably 50 µM; for the cytarabine, this concentration is generally greater than or equal to 40 µM, in particular comprised in the range 40 µM - 600 µM, preferably 40 µM; for the 6-thioguanine, this concentration is generally greater than or equal to 50 µM, in particular a concentration comprised in the range 50 µM - 600 µM, preferably 100 µM.

Said preferred concentrations are the CBCT values corresponding to the minimum concentration under standard conditions required to eradicate tumor cells in culture (minimum functional concentration), but could also be higher. In fact, by virtue of the protective action of the class A compound on the normal cells, such concentration may be increased up to 10-20 fold the mentioned minimum concentration thereof.

In this connection in fact generally the combination CBTC-TC can be varied by a person skilled in the art on the basis of the above information and of the state of the art in function of the desired eradicative effect. In particular it can be increased in an extremely remarkable manner both in the *in vitro* or *ex vivo* treatment, but above all in the former.

With regard to post-treatment, as reported above it generally includes the washing out of at least the administered class B compound from the culture, according to the above mentioned washing step.

Washing step time (WST) *in vitro* depends on the renewal rate of the medium and on the capability of the normal cells to catabolize the class B compound. Accordingly it can be generally selected from a range between 1 and 48 hours. Preferably said washing step is carried out for a time greater than or equal to 3 hours. Also in this case however it could be varied according to the characteristics of the cell type and the class B compound. Both in presence and in absence of the washing step after the interruption of the treatment with the class A and B compounds, generally the recovery of cell proliferation is allowed for a variable time, that can be calculated *in vitro* according to the experimental purpose, and *in vivo* according to the desirable tissue recovery.

The minimum estimate of the protection provided by the method of the invention in the embodiment wherein the normal cells are protected is of about 500/1000-fold.

A further preferred embodiment is that in which the sequence of the above mentioned treatments (pretreatment, combined treatment and post-treatment) is repeated in the colture twice or more, each time with the same modalities or with different modalities in function of the experimental purpose.

A further object of the present invention is the use of a protective compound having the capability of
- reversibly inhibiting cytodieresis of normal cells,
- not inhibiting the biological action of a chemotherapeutic compound, which chemotherapeutic compound has the capability of
   - exerting a cytotoxic action toward actively proliferating cells, but
   - not affecting survival and proliferative potential of interphase cells,
for the preparation of a medicament for protecting normal cells from the eradicative action of said chemotherapeutic compound in a treatment of a tumor form having an inactivated p53 pathway.

Such a use is the application *in vivo* of the above *in vitro* and *ex vivo* methods.

In preferred embodiments directed to the protection of all the normal cells and to eradication of all the tumor cells, the pre-treatment and/or the post-treatment as above defined, are also to be carried out.

In particular pretreatment *in vivo* comprises the administration to said subject in need thereof the protective compound as defined above, the administration of said protective compound resulting in the arrest at interphase of at lest part of said normal cells. The post-treatment *in vivo* includes a washing step wherein
- after interrupting the administration of said chemotherapic compound, said chemotherapic compound is to be washed off subject tissues, the administration of said class A compound being to be maintained.

A first advantage of the *in vivo* use of the present invention is that a complete protection of many normal cells, arrested in intermitotic phase, from the eradicative action of the class B compound, is thereby obtained. Said cells in fact resulted to be protected effectively and reversibly in order to allow the recovery of their physiological proliferative ability. The complete eradication of the tumor cells by the patient tissues is therefore obtainable as said eradication treatment can not affect normal cell arrested in the intermitotic phase, if class B compounds as here defined are used.

A second advantage of the *in vivo* use of the present invention is that both protection of normal cells and eradication of tumor cells are in any case controllable as functions of the parameters of each treatment as herein defined.

In particular the PT and CAP of the pretreatment of said *in vivo* method are the ones allowing the complete protection of all the proliferating normal cells present in the patient tissues, according to the therapeutical approach followed.

With specific regard to PT, like the *ex vivo* treatment, the preferred time is the time required by all the physiologically proliferating normal cells in the human body before they arrest their growth. Such time can be predetermined for all cell types, since the cell cycle time *in vivo* is well standardized. Like in the *ex vivo* and *in vitro* methods above, this time ranges between 12 and 96 hours.

However these times in the different application may be lengthened or shortened, depending on the different features of proliferating normal cells to be protected.

As to the CAP parameter, it is that required for the drug plasmatic concentration to reach a level sufficient to arrest proliferating normal cells to be protected at the intermitotic phase, in the different target normal tissues. In this case as well, such a value can be easily determined by those skilled in the art for each single class A compound depending on the administration mode preselected according to the knowledge of the state of the art and to the rules set by the present invention.

In general, such level is comprised within the above-mentioned range (1 nM -40 µM). However, also in vivo for each compound the CAP value corresponding to the minimum concentration required for reaching the arrest of normal cells at the intermitotic phase in the tissues to be protected (minimum functional concentration), like the ones indicated above, should be considered as preferred.

The combination CAP-TP can be varied in each case by a person skilled in the art on the basis of the above information and of the state of the art in function of the desired protective effect.

With regard to combined treatment, analogously to the pretreatment also the CT CACT and CBCT are the ones allowing the complete eradication of the tumor cells in the patient tissues, according to the therapeutical approach followed.

In particular, CT can therefore be defined as the minimum time needed by the tumor cells having inactive p53 pathway to complete at least one cell cycle, but it is preferably the time needed by the tumor cells having inactive p53 pathway to complete two or more cell cycles, in order to eradicate even those tumor cells that might have survived after the first cell cycle in the presence of class A and class B compounds. Also *in vivo* the two tumor cell cycle time constitutes no top limit to the CT time, which can be further increased (see above).

In addition, also CT shorter than one cell cycle time may be required in some eradication protocols (e.g. in order to obtain a more gradual eradication of the tumor and thus avoiding potentially harmful inflammatory reactions due to a too high rate of tumor cell death).

Concerning the concentration of class B compound (CBCT) it is a function of the combined treatment time, the target cell type and the half-life *in vivo* of the administered class B drug.

Also *in vivo* it can be 100nM - 800 µM in the patient tissues. However, by virtue of the protective action of the class A compound, such concentration may be increased up to 10-20 fold the mentioned minimum concentration thereof.

This possibility, (proper of the *in vitro* and *ex vivo* treatment, where however the treatment time and the concentrations of the adopted drugs are quite easily controlled by the operator) is most important in the in vivo treatment, in which the variations entailed in the blood transport of the drug and in the half-life thereof in the blood and in the tumor tissues need to be taken into account. For instance, in fact all the nucleoside-analogue chemotherapeutic drugs are generally known to have a very short half-life *in vivo* (about 1-3 hours).

Moreover, transient plasmatic concentration peaks are usually detected following the administration of nucleoside-analogue drugs. Therefore, in order to obtain a constant concentration at the cell level a prolonged treatment should be provided, presently entailing heavy side-effects. Instead, following a treatment with a class A compound, an *in vivo* perfusion of the chemotherapeutic drug (class B compound) could be carried out.

In this connection, like in the *in vitro* and *ex vivo* treatments) both the administration time of the class B drug (corresponding to the CT time in the method) and the concentration itself of such class B drug (corresponding to the CBCT concentration in the method) can be increased *in vivo* in an extremely remarkable manner.

This fact allows to attain also *in vivo* the suitable concentrations of class B chemotherapeutic agents drug and/or the administration time required for the eradication of all the tumor cells in the starting context.

With regard to post-treatment, the WST, *in vivo,* beside the cell capability of catabolizing the class B compound, is also function of the intrinsic hematic and/or tissutal half-life of the class B compound itself.

A further preferred embodiment is that in which the sequence of the pre-treatment, combined treatment and/or post-treatment are to be repeated, with the same modalities or with different modalities in function of the therapeutical purpose.

The *in vivo* use of the present invention can be used either for a systemic treatment or for a topical treatment. Furthermore, provided that target tumor cells have an inactivated p53 pathway, it can be applied to malignant tumor as well as to benign tumors such as the hyperproliferative lesions caused by papillomavirus infection. It can be also applied in the treatment of pathological infections caused by microorganism having an no p53 pathway and therefore no p53 function, and in the treatment of halopecia associated to standard systemic chemotherapy.

Accordingly, the present invention further relates to the use of a protective compound as defined in claim 14 for the preparation of a medicament for protecting normal cells from the eradicative action of a chemotherapeutic compound in a treatment of a pathological infection caused by microorganisms displaying no p53 function.

In preferred embodiments, a pre-treatment and/or a post treatment as defined in the method for protecting proliferating normal cells in a treatment of tumor forms above, are to be included.

The treatments of the use can be carried out via systemic and/or topical administrations. Microorganisms having no p53 function are any prokariotic and eukariotic microorganisms having no p53 pathway, which a pathological infection is associated to. A subject in need thereof can be a subject of any animal species, preferably human beings.

Object of the present invention according to what set forth above is also a use of a protective compound as defined in claims 14 for the preparation of a medicament for preventing and treating alopecia associated to a systemic administration with a chemotherapeutic compound in a treatment of cells having an inactivated p53 pathway.

In this case, a preferred form to delivery the class A compound is a topical administration.

Analogously to the uses above, in preferred embodiments a pre-treatment and/or a post treatment as defined for protecting proliferating normal cells in a treatment of tumor forms above, are to be included.

Object of the present invention is also a pharmaceutical product comprising a therapeutical effective amount of a protective compound (class A compound) as above defined, a therapeutical effective amount of a chemotherapeutic compound (class B compound) as defined above, in a pharmaceutically acceptable vehicle, carrier or auxiliary agent

Further object of the present invention is a pharmaceutical composition suitable as an adjuvant in particular in a treatment of a tumor form having an inactivated p53 pathway, in particular the forms having a low proliferating potential, of an hyperproliferative lesion caused by papillomavirus and of halopecia associated to systemic chemotherapy comprising a therapeutical effective amount of a protective compound (class A compound) as above defined, optionally a therapeutical effective amount of a chemotherapeutic compound (class B compound) as defined above, and a pharmaceutically acceptable vehicle, carrier or auxiliary agent.

A therapeutical effective amount of the protective compound according to the present invention is an amount whereby CAP and/or CATC concentration as indicated above in the methods of the present invention are obtained in target tissues. In fact said CAP and CATC concentrations allow the protective compound of the present invention to exert its therapeutical effect in a target tissue which is an adjuvant effect consisting typically of cytoprotection.

A therapeutical effective amount of the chemotherapeutic compound according to the present invention is an amount whereby CBTC concentration as indicated above in the methods of the present invention is obtained in target tissues. In fact said CBTC concentration allows the chemotherapic compound of the present invention to exert its therapeutical effect in a target tissue which typically is cytotoxicity.

In this connection such a therapeutical effective amount of the protective compound and chemotherapeutic compound, suitable as adjuvants and effective agents in the different treatments evidenced above, can be different in the pharmaceutical compositions of the present invention.

In addition both the therapeutical effective amount of the protective compound and the therapeutical effective amount of the chemotherapeutic compound of the present invention can vary among systemic and topical administration, depending also on the route of administration and the vehicle and/or carrier used.

A person skilled in the art can however derive such amounts on the basis of the state of the art and the teaching herein disclosed.

In the case a chemotherapeutic compound is included the composition is manufactured according to state of the art so that pharmacological release of chemotherapeutic compound is preferably retarded with respect to the pharmacological release of the protective compound.

Preferably the protective compound is selected from the group consisting of cytochalasins, with the exclusion of cytochalasin B, jasplakinolides, chondramides, isoindolinones, and the latrunculines, specifically can be cytochalasin D, dihydrocytochalasin B, jasplakinolide, chondramide B and/or latrunculin B.

Preferably the chemotherapeutic compound is selected from the group consisting of folate inhibitors, nucleoside analogues, nucleotide syntesis inhibitors, vinca alkaloids, taxanes, colchicine derivatives, podophillotoxin derivatives, and topoisomerase inhibitors, specifically can be trifluorothymidine, cytarabine, 6-thioguanine, 6-mercaptoputrine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, and/or 9-amino-S(20)-camptothecine.

A pharmaceutically acceptable carrier, vehicle or auxiliary agent for the pharmaceutical composition of the present invention is any carrier, vehicle or auxiliary agent known in the art to be compatible to the above mentioned described compounds.

Object of the present invention is also the following kits:
- a kit of parts for selectively eradicating cells having an inactive p53 pathway and selectively protecting proliferating normal cells comprising:
   - a protective compound as above defined (class A compound or drug);
   - a chemotherapeutic compound as above defined(class B compound or drug)
   as a combined preparation for simultaneous separate or sequential use in the *in vivo* and/or *ex vivo* therapy of the tumor forms having an inactive p53 pathway (including benign forms like an hyperproliferative lesion caused by papilloma virus), preferably the ones having a low proliferation potential.
- a kit of parts for selectively eradicating cells having no p53 function and selectively protecting proliferating normal cells comprising:
   - a protective compound as above defined (class A compound or drug);
   - a chemotherapeutic compound as above defined(class B compound or drug)
   as a combined preparation for simultaneous, separate or sequential use in the therapy of a pathological infection associated to a microorganism having no p53 function;
- a kit of parts for selectively eradicating cells having an inactive p53 pathway, or no p53 pathway and selectively protecting proliferating normal cells comprising:
   - a protective compound as above defined (class A compound or drug);
   - a chemotherapeutic compound as above defined(class B compound or drug)
   as a combined preparation for simultaneous, separate or sequential use in the method for protecting proliferating normal cells in an *in vitro* colture of the present invention.

In the kits of the present invention the protective compound preferably is selected from the group consisting of cytochalasins, with the exclusion of cytochalasin B, jasplakinolides, chondramides, isoindolinones, and the latrunculines, specifically can be cytochalasin D, dihydrocytochalasin B, jasplakinolide, chondramide B and/or latrunculin B.

Preferably the chemotherapeutic compound in the kits of the present invention is selected from the group consisting of folate inhibitors, nucleoside analogs, nucleotide syntesis inhibitors, vinca alkaloids, taxanes, colchicine derivatives, podophillotoxin derivatives, and topoisomerase inhibitors, specifically can be trifluorothymidine, cytarabine, 6-thioguanine, 6-mercaptoputrine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, and/or 9-amino-S(20)-camptothecine.

The protective and chemotherapeutic compounds of the kits of the present invention can be included in the kit together with a compatible vehicle, which in the kits for medical use is also pharmaceutically acceptable in a protective composition and therapeutic composition respectively.

A compatible vehicle according to the present invention is any vehicle known in art to be compatible with the compounds as above defined.

Object of the present invention are further the use of the C3H10T1/2 cells, or of cells derived therefrom according to the techniques known in the art to this purpose, to derive methods to eradicate tumor cells having an inactive p53 pathway from coltures comprising said tumor cells having an inactive p53 pathway and normal cells, and in particular a method for a treatment of a tumor form having an inactive p53 pathway.

Object of the present invention is further the use of the method and of C3H10T1/2 cells, or of cells derived therefrom according to the techniques known in the art to this purpose, for identifying protective compounds and/or chemotherapeutic compounds as defined above.

The invention will be better described with the aid of the annexed figure.

### Description of the Figure

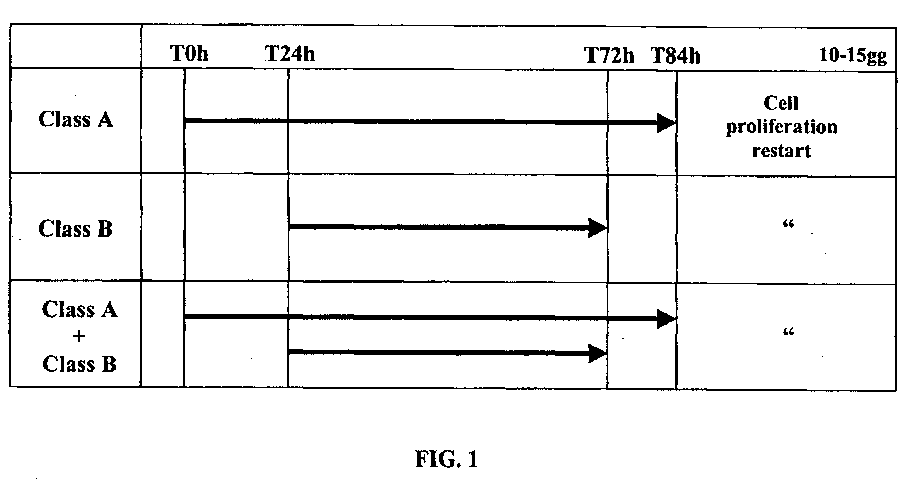

Figure 1 shows the time chart in tabular form of the method of the present invention in a preferred embodiment thereof effected on a system of C3H10T1/2 and C3H10T1/2*v-src*/*v-myc* cells.

The terms T0h, T24h, T72h, T84h indicate the times of the method expressed in hours; in particular, T0h corresponds to 0 hours, T24h to 24 hours, T72h to 72 hours and T84h to 84. hours.

In line 1, the time of the treatment effected with only the class A drug from time T0 to time T84 is reported.

In line 2, time of the treatment effected with only the class B drug from time T24 to time T72 is reported.

In line 3, times of the method according to the present invention as applied in its preferred embodiment to the above-indicated cells are reported: in particular, it is highlighted how the treatment with the class A drug is effected from the time T0 to the time T84, and the treatment with the class B drug overlaps with the class A drug treatment from the time T24 to the time T72. The time T0-T24 is the PT time, the time T24-T72 is the CT time, the time T72-T84 is the WST time, the time 10-15 days is the time for allowing proliferation of normal cells after treatment.

### Detailed Description of the Invention

A main feature of the in vitro methods and Medical Uses of the present invention is the relevant applicability to any tumor form having an inactive p53 pathway. Such a feature has been derived by several assays directed first to verify the molecular causes of the binucleation/multinucleation phenotype in normal/tumor cells.

In the first instance, therefore an assay series extensively described in example 1 has been carried out to verify whether the determination of such status could have been ascribed to the loss of the functions associated to one or more "tumor suppressor genes" (hereinafter also referred to as TSG) (13).

Such assays have been carried out on cells knock out (genetically null) for single TSG functions, as the relevant results are directly significant for the tumor forms to which they are normally associated (14).

The TSG functions investigated are the most relevant ones: Rb (retinoblastoma), p21 and p53 genes. In the relevant knock out cells, the function does not exist, therefore the obtained data can be ascribed to the loss of that specific function.

The nuclei number state of the knock out cells after said assays (see example 1) indicates that p53 function has a central role in the determination of the multinucleation state in the tumor cells, while Rb and p21 knock-out do not (the relevant knock out cells retain in fact the binucleated status). Growth arrest at the binucleate stage in the absence of p21 or pRb strongly suggests that cytokinesis blockade triggers a novel growth inhibitory pathway, distinct and different from that associated to growth arrest induced by microtubule destabilizing agents and X-ray irradiation, known to require both p21 and pRb (15).

Further assays on p53 knock-out cells, reconstituted with a. temperature-sensitive allele of p53, while confirming the previous data (see example 1) have been also indicative of p53 function as a check-point "switch" for the binucleation/multinucleation state in cells. p53 has in fact been proved to be necessary and sufficient for the establishment of such a "differential status" between tumor cells and proliferating normal cells.

Following these assays carried out on murine cell systems, the validity of relevant results has been confirmed and extended to human cell lines, i.e., those of greater interest. The cell lines used were human lung fibroblasts (LHF), either normal or transformed by transfection with two papilloma virus oncogenes (E6 and E7), capable of binding to and of functionally inactivating the p53 and the pRb gene products, respectively.

The assay series described in example 2 extended the validity of the murine cell data to human cell lines, thus supporting the conclusion that p53 regulates a cytodieresis check-point, the loss thereof being a condition sufficient for the induction of a multinucleated state in cells where cytokinesis is blocked.

The inactivation of p53 pathway is a condition detectable in most known tumor forms (14). Most of them in fact not only have, but are characterized by, an inactive p53 function. However even in unknown tumor forms or in tumor forms for which no information regarding p53 pathway is available, in view of the above results, an inactive p53 pathway is *a priori* indicative of capability of multinucleation.

In order to verify whether and to what extent such a state could be used as a "differential status", an *in vitro* model system, for verifying compounds and conditions which could be relevant to the *in vivo* situation has been derived.

### Derivation of a model system

In order to derive such a system which, as above-highlighted in the overview of the state of the art, was unavailable in the art, a considerable number of assays has been carried out.

In order to be subject to quantitative tests apt to demonstrate and validate the model, a novel system was required, made up by:
a) normal cells, displaying phenotypic stability in culture and maintaining the arrest at the binucleate stage following the administration of cytokinesis inhibitors;
b) an isogenic tumoral counterpart, also displaying phenotypic stability, but capable of undergoing multinucleation in the presence of cytokinesis inhibitors (i.e. with a p53 function inactivated or bypassed)

As a consequence of the poor growth properties of natural primary cells (not useful *in vitro* in the experimental approaches directed to clonogenic potential measurement), commonly used cell lines have been screened.

The majority thereof however demonstrated to be naturally prone to evolve toward a transformed state (with the consequent loss of capability to stop at the binucleate stage).

The C3H10T1/2 cells (mouse fibroblasts) were eventually selected, as being in fact relatively stable, displaying clonogenic potential in culture, and exhibiting the capability to undergo binucleation following the administration of the cytochalasin D (CD) used in these assay series (and in the preceding ones reported in example 1 and 2) to inhibit cytokinesis in normal cells.

Then, the tumoral counterpart of C3H10T1/2 had to be derived, which implied the overcoming of some problems.

The main one was consequent to the need of obtaining cells stably transformed and at the same time polyclonal in order to provide the highest measuring reliability for the above mentioned purpose (verifying that the binucleation/multinucleation is suitable as a "differential status"). The sole treatment ensuring stable and polyclonal tumor population for C3H10T1/2 cells, which are extremely similar to primary cells, is in fact cooperative transformation in sequence with two retroviral oncogenes, such as *v-src* e *v -myc* oncogenes.

In any case due to prejudices in art regarding the retroviral oncogenes (16), it was anyhow necessary to assay the multinucleative capability of the cells co-transformed with *v-src* and *v*-*myc* following the administration of cytochalasin D. The positive outcome of the relevant assay has provided also clear indication in that transformation in sequence with *v-src* and *v-myc* causes inactivation or bypass of the p53-dependent cytokinesis check-point.

In the light of this result, a model system has been derived, comprising cultures of either C3H10T1/2 normal cells or C3H10T1/2*v-src*/*v-myc* tumor cells cultivated alone, and a mixed one, made up by C3H10T1/2 and C3H10T1/2*v-src*/*v-myc* cells. The latter in particular has been devised so as to be mostly made by normal cells and by a lesser amount of tumor cells (akin to an *in vivo* situation).

Then, this system has been subject to an assay series directed to analyze cell nuclei number and the capability thereof of carrying out DNA synthesis, in the presence or absence of cytochalasin D.

In view of the positive outcome of such assay series the above system resulted validated.

### First definition of the in vitro Method and Use of the present invention

In order to verify then, the possible suitability of using the binucleation/multinucleation state as a "differential status", an assay series was carried out using CD and as an eradicative agent trifluorothymidine (3FT), a very strong, cytotoxic agent.

In all the three types of culture both cell types (normal and tumor) were kept in actively proliferating conditions in order to avoid distortions in the results due to the possible cell cycle arrest for causes independent from the compound administration.

The combined administration of said two compounds resulted in fact in a tumor cells eradication.

As a consequence CD and 3FT have been then administered at different dosages and times, having taken care to always carrying out the necessary verifies in order to find the most effective combination of the administered times/concentrations parameters.

The most significant assay series referring to the optimum parameter combination finally found for attaining the total eradication of the tumor cells and the effective protection of the normal cells, are extensively disclosed in examples 3 (qualitative assays) and 4 (quantitative assays).

In this way the method steps in the preferred embodiment were derived, wherein all the tumor cells are eradicated and most proliferating normal cells are protected, as above described in the summary of the invention (see also Fig. 1).

The choice of colony formation assay (that provides a direct indication of the clonogenic potential of the cells) as operative assay instead of other assays (as, e.g., the plated cell count) used in measuring the proliferative capability is consequent to the need of stringently verifying not only the induction, but also the reversibility of such a differential growth arrest "status" in normal cells.

In particular, concerning the pretreatment step, the corresponding duration of 24 hours indicated in the scheme of Figure 1 is slightly longer than the cell cycle time of the C3H10T1/2 cells used. Though the cytochalasin D concentration reported in this assay series is of 400 nM, yet even concentrations comprised in a range of 50 nM - 1.6 µM proved effective.

Concerning the combined treatment step, it has been carried out for 48 hours (time necessary by the C3H10T1/2*v-src*/*v-myc* tumor cells for the completion of two cell cycles, and, moreover, the minimum time required for the complete cell post-treatment killing with the class B drug alone).

The cytochalasin D concentration has been kept constant with respect to the pretreatment. The administered 3FT concentration was of 40µM, yet even concentrations comprised in a range 10 - 800 µM proved useful.

Concerning the post-treatment step: the washing step has been carried out for a time of 12 hours, time calculated in function of (specifically, it is inversely proportional to) the catabolic rate of the class B drug.

Concerning instead the proliferation restart of the normal cells, as it is by definition variable, in the specific case of a colony-forming assay *in vitro,* it has been set as the time required to obtain visible colonies (that varies according to the pre-selected cell type).

In the assay series instead directed to obtain total eradication of the tumor cells and the total protection of many normal cells it is the time required for a correct reforming of a normal cell monolayer, and for the appearance of possibly visible (i.e., having a dimension of at least 2 mm) tumor cell foci (see example 4).

In this regard, in order to attain a reliable result, such a time has been - and should always be - calculated on the basis of the synchronization of the test control group. The different proliferation restart time of the control cells shall accordingly be taken into account. In fact, the latter cells have a shorter restart time with respect to the treated cells, that, having been arrested, have lengthier colony-forming times with respect thereto, according to the above-stated standards.

More specifically, taking into account the times of the assay control group, such a time, in the assays carried out on the system of the present invention, is comprised in a range of 7 to 15 days. In particular, for instance, when the control cells (both normal and transformed) have a 7-day post-treatment time, the cells treated with cytochalasin D have a 10-day time, and so on.

From the data it is evident that, while normal and transformed cells are equally killed by the 3Ft treatment, the CD/3FT combined treatment displays a selectivity index of at least 500 fold toward transformed cells: i.e. normal cells have became at least 500 fold more resistant to the 3FT lethal effects as a consequence of being protected by the CD administration, whereas transformed cells remain fully susceptible.

### Class A compound definition:

In outcome of the assays above, while obtaining accurate indications referred to the combined use, positive indications have been at the same time obtained on the suitability of cytochalasin D and 3FT for the selective eradication of tumor cells having an inactive p53 pathway.

In particular CD as the compound inducing the protective, "differential status" and 3FT as the eradicating agent. In this connection, in order to verify the relevant equivalent compounds (herein defined class A and class B compound respectively), in a first instance an assay series has been carried out to verify which features were responsible for the capability of inducing such a status and eradicating the tumor cells therein respectively.

With regard to CD, it has been first hypothesized the relevant properties on the cell system of the present invention to be ascribable to:
1. the capability of inhibiting cytodieresis of normal cells;
2. reversibility of such an inhibition and
3. capability of non-inhibiting the biological action of a second compound (hereinafter also referred as class B compound or drug).

In principle, the three properties above described represent the crucial features that any drug must display in order to be used in the protection of normal cells. In fact,
- drug-induced inhibition of cytodieresis activates a checkpoint that leads to growth arrest in normal cells, but not in tumor cells with a disrupted p53 pathway;
- drug-induced growth arrest has to be fully reversible so that normal cells can resume proliferation and perform all their qualitative and quantitative functions;
- should the drug affect also the intracellular transport of the secondary chemotherapeutic drug (i.e. class B compound) over an acceptable threshold, then tumor cell killing by the class B drug will be accordingly diminished in the combined treatment.

Such hypotheses have been verified in two assay series directed, first of all, to the verification of the above mentioned features of CD.

Regarding the first assay series extensively reported in example 5, the first two features (capability of reversibly inhibiting cytodieresis in normal cells) have been assayed.

The third feature has been verified with a further assay series, in particular consisting of a colony-forming assay in a system exclusively formed by the tumor cells has been carried out, wherein the effects of the cytochalasin D administration have also been compared to ,those of the administration of the cytochalasin B (CB).

In outcome to such an assay, extensively described in example 6, it is observed that while CB clearly interferes with 3FT toxic effects ensuring a certain degree of survival to the tumor cells, CD allows no survival whatsoever of such cells (see table 6). 3FT administration, in fact combined with the CD administration, carried out according to the parameters of the complete and maximally selective eradication, determines the complete eradication of the cultured tumor cells, whereas the CB administration under the same conditions merely entails a partial eradication. Such a partial eradication as due to the above mentioned interference and not to a specific setting of the above mentioned parameters, is accordingly not controllable. As a consequence such an assay not only confirms the third above mentioned features to be ascribable to CD, but also said the absence of said feature in CB. The corresponding non suitability of CB to obtain an eradication selective, complete and controllable, is a first observation supporting the notion that such a feature is a necessary condition, in conjunction with the other two features for a compound to be able to induce such a differential status.

In order to verify further whether having said features is a necessary and sufficient condition for a compound to be a class A compound, a subsequent assay series have been carried out in order:
- in a first instance, to verify the said features to be proper of some candidate compounds selected from the ones known to be capable to induce multinucleation;
- after that, to verify the suitability of the positive compounds in the method above described in the form which shall results in the total eradication of tumor cells and maximal protection of normal cells.

In this connection, the same assay series carried out for CD and described in example 5 and 6, have been carried out with other candidate compounds (see examples 7 and 8). In particular, members of the latrunculines, the jasplakinolides and the chondramides have been assayed. All the tested compounds, and in particular latrunculin B, jasplakinolide, chondramide B, show results in all equivalent to that of cytochalasin D.

In particular, in the model system jasplakinolide and chondramide show an extremely reversible action, to such an extent that in tumor cells these substances interfere with the polymerization of the actin cytoskeleton, but without any stable cytodieresis inhibition. However, this effect does not impair the class B compound effectiveness onto the tumor cells, nor the effectiveness of the same protective action thereof onto the normal cells.

When subject to a tritiated thymidine uptake assay as extensively shown in example 8, they all show the capability of not inhibiting the ³H-thymidine uptake.

These data suggest that positively identified class A compounds do not appreciably interfere with intracellular uptake of nucleosides, including the nucleoside analog belonging to class B as here defined. This example is meant not to restrict the features of class B compounds, but to illustrate how interference by a class A compound on a class B drug can strongly reduce the efficiency of tumor cell eradication.

In outcome to said assay series, all these compounds have been assayed in the system under the condition for the complete eradication of the tumor cells in culture.

The positive outcome of said assays corresponding to the assays extensively disclosed in examples 3 and 4 confirms, on one hand the suitability of these compounds in the method of the present invention, on the other hand the above mentioned features to be the necessary and sufficient ones for a compound for being capable of inducing the "differential status", and therefore a class A compound according to the present invention.

Chondramides, jasplakinolides and latrunculines have been proved to be therefore class A compounds, that can be used for the same times disclosed for the cytochalasin D, and in a concentration range of from 1 nM to 40 µM.

In the light of these tests, the CAP and CACT values to be assigned to several class A compounds as the concentration range yielding the cytodieresis arrest, and, within said concentration range, the value of the minimum concentration required for attaining this effect, forming the preferred embodiment, has been determined. These values are those reported in the summary of the invention.

As a consequence of all these assays, from the analysis of the specific characteristics of the positive compounds, the class A could be defined as a functional class of compounds useful in the in vitro Method and Use according to the present invention.

In this connection a positive outcome of an assay series of examples 7 and 8 as carried out with a candidate compound automatically defines it as a class A compound.

### Class B compound definition

Analogously to what reported for class A compound and in particular for cytochalasin D, the features characterizing the action of 3FT have been first hypothesized and then assayed. Necessary and sufficient features for defining a compound as belonging to class B according to the present invention are the capability of:
- having a cytotoxic action toward actively proliferating cells and
- not affecting proliferative potential and viability of interphase cells.

Said features are in fact proper of 3FT as resulting from the assays reported in example 3 and 4.

While many drugs and compounds are known that efficiently kill replicating cells, the crucial feature of the eradication agent to be successfully used in the combination treatment as here defined is that of being devoid of any adverse activity on interphase cells. Such a property is crucial as it influences directly the efficiency of normal cell protection by class A compounds.

The same features have been assayed in candidate compounds which have been themselves subjected to the example 3 and 4 assays, instead of 3FT.

Of particular relevance are the assays carried out with two drugs commonly used in chemotherapy: the Ara C (a.k.a. cytarabine) or the 2chlorinedeoxyadenosine (2ClAd, o.k.a. Cladribine), known for the action thereof on highly proliferating cells, extensively described in example 9.

The relevant outcome is that in both cases (Ara C and 2ClAd) a drastic decrease, if not altogether the absence of the transformation foci, and the concomitant reforming of the normal cell monolayer was observed (see example 9).

However, in the case of the combined treatment with the 2ClAd, the normal cell monolayer reformed more slowly (a 14-day proliferation restart time was necessary, versus the approximate 7-day time of the method carried out with Ara C or 3FT) nearly doubling the restart time with respect to the other two tested drugs.

Moreover, such monolayer cells have a senescent appearance, and the monolayer saturation density (number of cells per surface unit) is remarkably lower with respect to the other two cases.

These data, related to the time of the proliferation restart phase and to the morphology of the normal cells after the whole treatment, points to the existence of a certain toxic effect of the 2ClAd also on the normal cells in the combined treatment.

Therefore, a further assay series has been carried out as to verify the absence of cytotoxicity on cells in the intermitotic phase, of the candidate compounds extensively described in example 10.

Such assays were directed to analyze the antimetabolite toxicity on cells arrested in the G0/G1 phase (the cell cycle stage at which normal cells are arrested by the administration of class A compounds) by confluence or serum deprivation. Observing the number of surviving cells, and the number of those also proving capable of reentering the cycle, the results of the example 9 assays have been confirmed. In fact, in this case as well, out of the 5 antimetabolites utilized, the 3 positive to the test disclosed in example 9(3FT, Ara C, 6ThG) proved wholly functional.

The sole 2ClAd, that caused toxicity problems in the previous test according to example 9, and doxorubicine, assumed to be potentially functional according to the art, resulted instead in a complete inhibition of the S phase entry. Moreover, doxorubicine exhibited also a strong cytotoxic effect.

Thus, in outcome of the above assays Ara C and 6ThG have been considered eligible as class B compounds, whereas 2ClAd and doxorubicine, due to their toxicity were discarded.

In addition to that in outcome to said assays the above mentioned features have been confirmed as the sufficient and necessary condition for a compound to be considered a class B compound.

Generally, all the compounds having a toxicity not substantially altering the cell vitality and the proliferation potential of cells in the intermitotic phase and the cell type-specific physiology in fact are class B compounds.

In this connection a positive outcome of an assay series, such as that described in examples 9 and 10, with a candidate compound automatically defines it as a class B compound.

In light of these assays, in order to check the validity of the utilized concentrations, and, above all, the possibility of increasing the latter and/or the administration time of the class B drugs, an assay series extensively described in example 11 has been carried out, wherein the consequences of class B drugs at various concentrations, up to a 10-20 fold increase thereof, has been assayed.

Of course, this would have constituted a further advantage entailed in the application of the in vitro Method and use of the invention, as it is evident that most tumors would certainly be eradicated by treatments with chemotherapeutic agents drug concentrations increased 10-fold with respect to those actually used. To date, this remains unfeasible as the administrable chemotherapeutic agents drug dosage is limited by the toxicity thereof.

The outcome of the assays series described in example 11 is that trifluorothymidine starts to be toxic at 800 µM, whereas the Ara C toxicity starts between 200 and 400 µM.. Of course, the two really toxic drugs already excluded from class B (2ClAd and Doxorubicine) utilized as positive control exhibit strong toxicity already at the minimum functional concentrations.

From these results, all the more so when compared to those related to the two really toxic - and anyhow non-class B - drugs, it can be stated that the chemotherapeutic drug concentration may be increased up to 10-20 fold by virtue of the chemoprotection selectively provided to normal cells by the use according to the present invention. Although a certain toxicity was detected after increasing the concentration with the compounds included in the class B as defined, toxicity remains at very low levels as compared to that of 2ClAd and doxorubicine.

For the sole Ara C as a class B drug, a 6-fold dosage increase begins to entail a relevant toxicity.

### Extension to the human system of the validity of obtained results

As reported above the model system has been selected in order to reproduce the *in vivo* and the *ex vivo* conditions as faithfully as possible. As a further confirmation of their applicability to the pathological situation in the human system *in vivo*, the assay series extensively described in example 12 has been carried out.

The relevant outcome confirms that the administration of the class A compounds on human tumor (Lipari glioblastomas, T97, U973), and normal (LH and DH fibroblasts) cells determines the same effects recorded on the model system cells CH310T1/2*v-src*/*v-myc* and C3H10T1/2, respectively.

Hence, the complete validity of the combined use of the drugs of the two classes as above disclosed on the human system as well has been fully demonstrated.

### Definition of the in vitro Method and Use of the present invention

In view of the results obtained in outcome of the assays above eventually class A compounds (in particular cytochalasin D, dihydrocytochalasin B, jasplakinolide, chondramide B and latrunculin B) and class B compounds (in particular trifluorothymidine, cytarabine, 6-thioguanine) have been then administered at different dosages and times, to the cell systems of the present invention having taken care to always carry out the necessary verifications in order to find the functional and effective combination of the administered times/concentrations parameters. Uses and in vitro methods including combined treatment only, and combined treatment together with pretreatment and/or post treatment as described in the summary of the invention have been therefore carried out, and the relevant parameters values derived.

Accordingly, the features of uses, kits and pharmaceutical compositions of the present invention as reported in the summary of the invention above, have been further derived.

A herein before general description of the present invention has been provided. Hereinafter, with the aid of the following examples, a more detailed description of specific embodiments thereof will be given, aimed at making the objects, features, advantages and operation modes thereof better understood.

### EXAMPLES

### Example 1: Binucleation/multinucleation status as a consequence of an inactive p53 pathway

In order to verify which molecular factors cause multinucleation in tumor cells and thereby identify tumor cells and tumor forms where binucleation/multinucleation differential status is applicable to, an assay series directed to verify such forms to be ascribable to the loss of one or more genes regulating cell functions, has been carried out. Such assays have been in particular carried out on cells deriving from mice knock-out for the sole Rb (retinoblastoma), p21 or p53 function. In each cell type, the relevant knocked-out function does not exist, therefore data obtained therefrom are automatically referable to the loss of that related function.

Each cell culture was plated on 35mm plates at a 1×10⁴cells/plate density, under the following conditions considered herein as standard conditions. Cells were grown on a plastic substrate (Falcon-BD), in a culture medium made of Dulbecco's modified Eagle's Medium supplemented with 2mM L-glutamine, antibiotics, 10% fetal bovine serum, at 37°C with 5% CO₂ and 90% humidity. The following assays have been therefore carried out.

In an initial time of 12 hours c.ca (step common also to the other assays herein reported) cells were allowed to grow up, but within a density allowing their proliferation, thus ensuring the utmost reliability of the assay result, that otherwise could be altered by cells arrested for reasons other than compound administration. After this first step the following administrations have been carried out.

In a first assay series 400 nM Citochalasin D for 72 h (T0-T72) has been administered to mouse embryofibroblast (MEF) cells knock out for Rb or p21 functions, respectively.

In a second assay series for 72 h (T0-T72) has been administered to MEF and dermal fibroblasts (MDF), respectively, as a wild-type control cells.

In a third assay series 400 nM CD for 72 h (T0-T72) has been administered to Balb C(10/1) (knock out cells for p53 function) at 32 °C and 37 °C respectively.

In a fourth assay series 400 nM CD for 72 h (T0-T72) has been administered to Balb C(10/1)-p53Val5 (knock out cells for p53 function, wherein a temperature sensible p53 mutant has been reintroduced), at 32 °C and 37 °C respectively.

Control untreated cells show less than 2% of binucleate (2N) or multinucleate (>3N) cells.

The administration was carried out on strictly proliferating cells. After treatment, cells were processed and analyzed.

The relevant results are reported in the following table 1

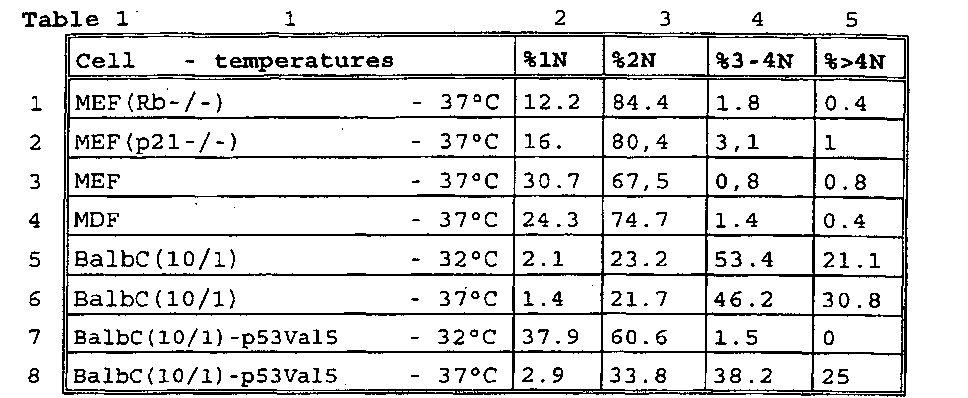

In lines 1 and 2, 3 and 4, 5 and 6, 7 and 8, results of the first, second, third and fourth assay series are respectively reported. In particular columns 2, 3, 4 and 5 percentages of mononucleate, binucleate, tri-tetranucleate, and >4 nuclei cells detected in outcome of said assays are respectively reported. In column 1 are instead reported the cell types used and the relevant assay temperatures.

The results of all these assays demonstrate that following administration of CD to both Rb^{-/-} and p21^{-/-} cells, as well as to MEF and MDF controls, cells arrest into a binucleate stage (see Line -4), proving that the absence of the Rb or p21 functions is not sufficient to induce multinucleation after the administration of cytochalasin D.

This implies that the Rb and p21 genes, which are known important factors repressing DNA endoreduplication after inhibition of mitosis, are not responsible for preventing multinucleation after the administration of CD and hence cytodieresis inhibition.

Results obtained with Balb/c(10)1 cells demonstrate instead the importance of p53 function(s) in multinucleation state determination upon cytokinesis blockade by CD (see in particular total number of cells having a nuclei number greater than 2 in lines 5 and 6).

The fact that the loss of such a function is sufficient for inducing multinucleation in cells is confirmed also by the assay carried out on Balb C(10/1) wherein a ts (temperature sensitive) p53 mutant has been introduced so that these cells display an inactive p53 protein (p53 inactive function) at 37°C and an active p53 protein (p53 active function) at 32°.

The results of said assays reported on lines 7 and 8 confirm that multinucleation after inhibition of cytokinesis is only observed in cells having inactive p53 function (see line 8 compared with line 7).

### EXAMPLE 2: Verification of extensibility to the human system of the p53-related data.

In order to confirm p53 function(s) to be the "switch" regulating CD-induced multinucleation state in cells (on when inactive and off when active), the same assays described in example 1 have been carried out on human lung fibroblast (LHF) transfected with 2 papillomavirus oncogens (E6 and E7). E6 is in fact an oncogene, the product thereof being capable of sequestering and inhibiting the p53 function, whereas E7 is another oncogene, the product thereof being capable of sequestering the Rb function. This assay series accordingly allows in fact to extend also to human cells the growth arrest, at the binucleate stage, by CD, as due to an active p53 pathway in normal cells, and at the same time it confirms the non-implication of Rb therein.

After the initial time of 12 h c.ca (see above) the following assays have been in particular carried out.

In a first assay series 400 nM CD for 72 h (T0-T72) or 0.11% DMSO for 72 h (T0-T72) as a control thereof, have been administered to control human lung fibroblasts (HF LXSN).

In a second assay series 400 nM CD for 72 h (T0-T72) and 0.11% DMSO for 72 h (T0-T72) as a control thereof, have been administered to human lung fibroblasts transformed with E6 (HF E6).

In a third assay series 400 nM CD for 72 h (T0-T72) and 0.11% DMSO for 72 h (T0-T72) as a control thereof, have been administered to human lung fibroblasts transformed with E7 (HF E7).

The relevant results are reported in the following table 2

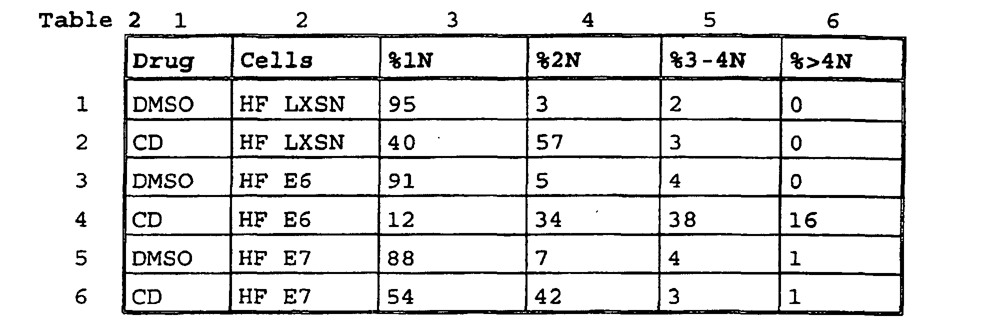

In lines 1 and 2, 3 and 4, 5 and 6, results of the first, second and third assay series are respectively reported. In columns 3, 4, 5 and 6 percentages of mononucleate, binucleate, tri-tetranucleate, and >4 nuclei cells detected in outcome of said assays are respectively reported. In column 1 and 2 are instead reported the administered drug and the assayed cell are reported.

The results above shows that while in the presence of CD the E6-transformed fibroblasts multinucleate (see line 4), the E7 ones do not (see Line 6), confirming the data of previous example 1 obtained with murine cells.

### Example 3: Normal cell protection by combined CD/3FT use: a qualitative evaluation

In order to verify pharmacological effects of combined CD/3FT use, formation of transformation foci in a mixed culture of normal and tumor cells has been assayed, the system including C3H10T1/2 as normal cells and C3H10T1/2*v*-src/v-myc or L cells as tumor cells, (in order to obtain data not limited to a specific cell system).

In each of the assays the above mentioned cells have been plated in 60mm plates. In particular C3H10T1/2 have been plated at the density of 10⁴ cells per plate, C3H10T1/2*v*-src/v-myc at the density of 5×10² cells per plate, and L cells at a density of 5×10² cells per plate.

Such a difference in density among normal and tumor cells reproduces in fact an *in vivo* neoplastic pathology situation wherein tumor cells are effectively less than normal ones, and allows also to obtain discrete and quantifiable transformation areas due the uncontrolled tumor cells growth. Culture conditions are the standard ones of example 1.

After the initial time of 12 hours c.ca (see above example 1), the following assays have been carried out on the two cell systems, C3H10T1/2-C3H10T1/2*v*-src/v-myc one and the C3H10T1/2- L cells one. First the following assay series have been carried out on C3H10T1/2-C3H10T1/2*v*-src/v-myc cell systems.

In a first assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a culture of C3H10T1/2 alone.

In a second assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a culture of C3H10T1/2*v*-src/v-myc alone.

In a third assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a mixed culture of C3H10T1/2 and C3H10T1/2*v*-src/v-myc.
After that, the following further assay series have been carried out on C3H10T1/2- L cells system.

In a fourth assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a culture of C3H10T1/2 alone.

In a fifth assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a culture of L-cells alone.

In a sixth assay series 0.11% DMSO and H₂O from T0 to T84, 400 nM CD from T0 to T84, 40 µM 3FT from T24 to T72, and 400 nM CD from T0 to T84 and from T24 to T72 also 40 µM 3FT, have been administered to a mixed culture of C3H10T1/2 and L-cells.

The administrations of sole CD and 3FT were directed to the measurement of the effects thereof on said cell systems, the combined CD/3FT administration to the measurement of the effects of the CD/3FT combined use, while the administration of DMSO has been made as a control.

The treatment time contraction from 72 to 24 h with respect to the assays carried out on primary cells of examples 1 and 2 is due to the fact that the latter have a longer cell cycle time with respect to that of the C3H10T1/2, therefore it was necessary to avoid any possible doubt related to the binucleate forms found after CD-treatment.

After each treatment cells were maintained in culture for a time necessary for detecting foci formation; the single experimental groups - e.g., the untreated (-/-) and the treated with CD only (CD/-) - have been examined at the same time.

At the end of each assay the cells were washed 2x with PBS and fixed 2x with 5% trichloroacetic acid at 4°C for 15 min, washed 2x with 70% ethanol and stained with 5% Giemsa solution for 30 min in order to clearly highlight the transformation foci and to maintain visible the normal cell monolayer, as previously described (17). The relevant results are reported respectively in sections A and B of the following table 3.

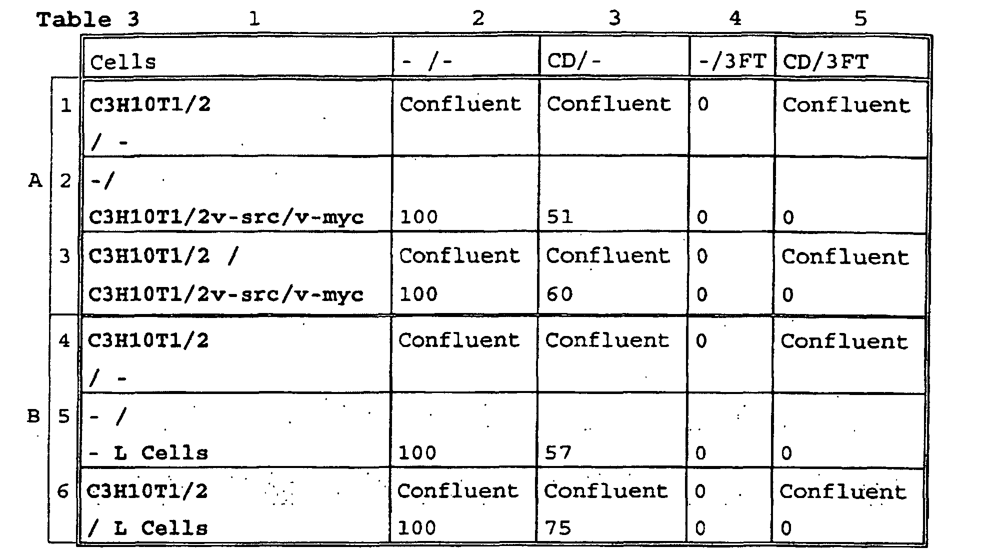

In section A results of the assay carried out on C3H10T1/2-C3H10T1/2*v*-src/v-myc cell system are reported. In particular in lines 1, 2 and 3 are reported the results of the first, second and third assay series respectively.

In section B results of the assay carried out on C3H10T1/2-Lcells system are reported. In particular in lines 4, 5 and 6 there are reported the results of the fourth, fifth and sixth assay series respectively.

In column 2, 3, 4 and 5 results of treatment with respectively the sole vehicles (DMSO for CD and H2O for 3FT), the sole CD, the sole 3FT, and CD/3FT, are reported. In column 1 there are reported the cell assayed.

The relevant obtained values, expressed as percentages of treated with respect to the untreated, identify the number of transformation foci detected after assay. The definition "confluent" indicates a completely formed monolayer in the plate. Each percentage value has been calculated on the mean data determined from duplicate plates.

Results obtained by these series of experiments on normal and tumor cells show a selective protective action of CD on normal cells corresponding to a complete and selective eradicative action of 3FT on tumor cells.

In particular in fact in the absence of any treatment normal cells in both system become confluent generating a monolayer, whereas the tumor cells originate a certain number of foci (value "100" is used as a reference) as shown in column 2.

When Cytochalasin D is administered during the whole treatment (24 h pretreatment, plus 48 h combined treatment, plus 12 h washing, up to a total of 84 h) only a slight decrease of the tumor cell-related value with respect to the control is detected at the end of the treatment (see column 3). This decrease is detected in either of the two tumor cell monocultures (lines 2 and 5) and in the two mixed cultures (lines 3 and 6), and is due to multinucleated cell status, which also causes a more difficult restart of cell proliferation.

When sole 3FT is administered, complete death of both cell types is obviously detected (see column 4)

Only in case a combined treatment of CD and 3FT is carried out, in particular following the steps described above (column 5), after a 7-day cell proliferation restart phase a complete and selective protection by CD of normal cells generating a confluent cell monolayer, vs. a complete and selective eradication of tumor cells is obtained (see column 5).

### Example 4: Normal cells protection by combined CD/3FT use: a quantitative evaluation

In order to quantify the effects of the combined CD/3FT use of the above example 3, the first, second and third assay series described in example 4 has been carried out on monocultures at a cell density allowing countable colonies.

The culture conditions, compounds concentrations, and treatments are the same described in example 3, with the exception of the cell system used have been for the first assay series a monoculture of C3H 10T1/2 cells at the clonal densities of 1×10³, 5×10³ and 1×10⁴, for the second assay series a monoculture of C3H 10T1/2*v*-src/*v-myc* cells at the clonal densities of 5×10², 1×10³ and 5×10³, and for the third assay series a monoculture of L cells at the clonal densities of 5×10², 1×10³ and 5×10³.

After each administration, cells were washed 2x with PBS and cultured for the necessary time for colony formation (as in example 2, the individual test groups were concomitantly fixed), and then processed as described in example 3

The relevant results are reported in the following table 4.

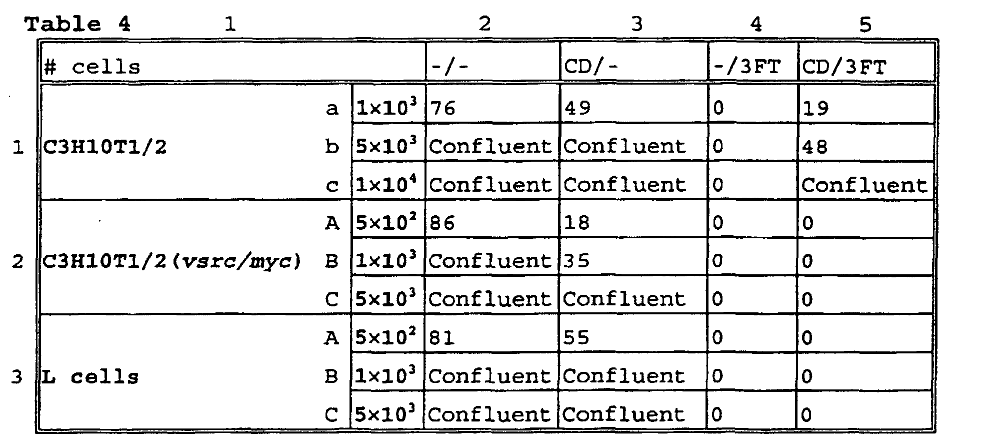

In line 1 results of the first assay series as carried out on C3H10T1/2 monoculture, at the clonal densities of 1×10³ (subline a), 5×10³ (subline b) and 1×10⁴ (subline c) are reported. In line 2 results of the second assay series as carried out on C3H 10T1/2*v*-src/*v*-myc monoculture at the clonal densities of 5×10² (subline a), 1×10³ (subline b) and 5×10³ (subline c), are reported.

In line 3 results of the second assay series as carried out on L cells monoculture at the clonal densities of 5×10² (subline a), 1×10³ (subline b) and 5×10³ (subline c), are reported.

In columns 2, 3, 4 and 5 results of treatment with respectively the sole vehicles (DMSO for CD and H2O for 3FT), the sole CD, the sole 3FT, and CD/3FT, are reported. In column 1 are instead reported the cell assayed and the relevant densities.

The relevant obtained values reported in table 4, correspond to the number of colonies detected after assay. The definition "confluent" indicates a complete cell monolayer in the plate. As in example 3 each value has been calculated on the mean data determined from duplicate samples.

Results of these assay series confirm the protection demonstrated by the assays of example 3(2), and moreover allow to validly quantify it.

In particular in case cells are allowed to proliferate administering DMSO and H₂O only for a time equal to the example 2 treatment one (84 hours), both normal and tumor cells give rise to confluent cultures at the two greater initial densities and to discrete, individual colonies at the lower (column 3, control data).

When instead CD only (column 4) 3FT only (column 5), or both (column 6), have been administered to each cell culture at the lower initial density, which is indicative of the clonogenic cell potential, the following values have been obtained.

For C3H10T1/2, the treatment with CD results in a decrease of the value from 76 to 49 significant of a certain toxic effect, the treatment with 3FT, the predicted value 0, and the combined treatment eventually results in a value 19 which is instead significant of the occurred protection.

For C3H10T1/2 *v*-src/*v-myc* the treatment with CD results in a decrease of the value from 86 to 18, significant of a CD toxic effect on tumor cells stronger than on normal cells, the treatment with 3FT the predicted value 0, and the combined treatment results this time in the value 0 which is significant of the complete absence of protective effects of such a treatment on this type of tumor cells.

For L cells the treatment with CD results in a decrease of the value from 88 to 55, significant of a CD toxic effect on tumor cells stronger than on normal cells, but weaker than on C3H10T1/2 *v*-src/*v-myc* tumor cells, the treatment with 3FT the predicted value 0, and the combined treatment results also in this case in the value 0 significant of the complete absence of protective effects of such a treatment also on this tumor cells types.

These data are confirmed and validated also by the assays carried out on the higher initial cell densities as, even starting from such higher values, the number of tumor cells foci obtained after the combined treatment is significantly always 0.

Comparing such values, separately obtained by quantitative measurements in different systems, a selectivity index of the chemoterapeutic agent could be estimated. In particular, when used alone, the chemotherapeutic agent has been demonstrated to be lethal for both normal and tumor cells, whereas, when combined with CD, in particular according to the hereto described protocol, 3FT resulted at least 500-1000-fold more effective in killing the tumor cells.

Eventually however the above results demonstrates further that 3FT while having a cytotoxic effects on tumor proliferating cells, does not have any toxic effect on arrested normal cells, as after stopping the combined treatment the latter are capable of proliferate till forming a confluent monolayer.

### Example 5: inhibition of cytodieresis and subsequent DNA replication in normal cells by cytochalasin D and reversibility thereof.

In order to verify cytochalasin D capability to inhibit cytodieresis and subsequent phases of cell cycle in normal cells, a series of colony-forming assay, wherein DNA synthesis arrest was detected by Bromodeoxyuridine (BrdU) uptake, has been carried out on C3H10T1/2 and in C3H10T1/2/v-src-/myc.

Each cell culture was plated on 35mm plates at a 1×10⁴cells/plate density, under the standard conditions described in example 1. After the initial time of 12 hours c.ca (see above example 1), the following assays have been carried out.

In a first assay series, CD for 48 h (T0-T48) and also BrdU in the last 24h (T24-48) for detecting percentage of cells capable of entering in S phase (herein also referred as %S), have been administered on C3H10T1/2 and in C3H10T1/2/v-src-/myc respectively.

In a second assay series CD for 48h (T0-T48) followed by a 24 h rest for allowing drug catabolysis, and then BrdU for further 24 h (T72-T96) for detecting %S, have been administered on C3H10T1/2 only.

In a control assay series thereof the sole BrdU and 0.11% DMSO, have been administered to C3H10T1/2 and in C3H10T1/2/v-src-/myc cells respectively, for 24h (T0-T24) as a control of the proliferation phase.

In each of the above assays CD and BrdU concentrations are the same, namely 400 nM and 20 mM respectively. The administration was carried out on strictly proliferating cells. The contemporaneous measurement of BrdU uptake and %S has ensured the highest stringency of results on DNA replication. Percentage of mononucleate, binucleate, tri/tetranucleate and with >4 nuclei resulting cells has been also measured. After treatment, cells have been processed and analyzed as reported in reference 18.

The relevant results are reported in the following table 5.

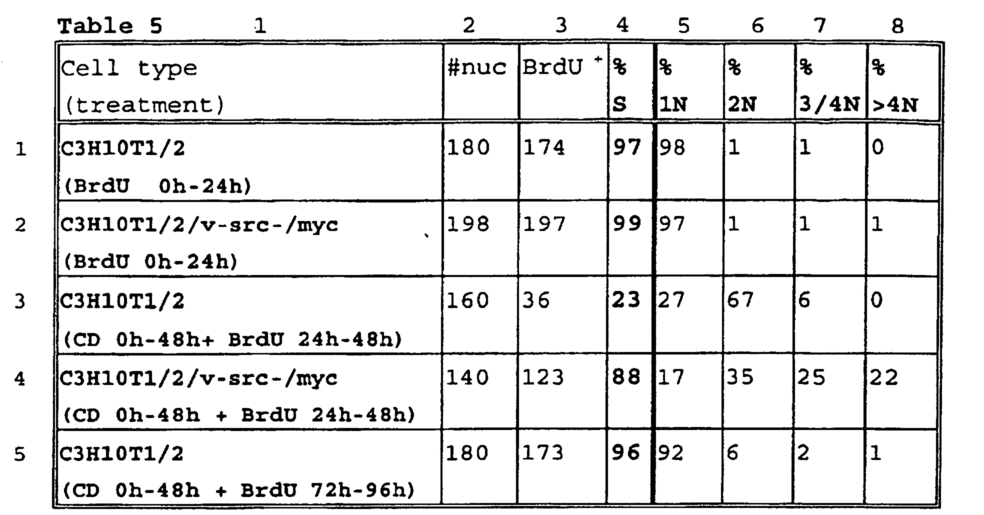

In lines 3 and 4 results of the first assay series are reported. In line 5 results of the second assay series are reported. In lines 1 and 2 there are reported the results of the control assay series.

In column 1, there are reported the cells assayed and the relevant treatment including the administration time in hours.

In column 2, 3 and 4, total nuclei number for each assay, number of nuclei having uptaken BrdU (therefore having synthesized DNA) in each assay, percentage of cells having synthesized DNA in each assay (data directly deriving from the latter two columns) are reported respectively. In columns 5, 6, 7 and 8 percentages of mononucleate, binucleate, tri-tetranucleate, and >4 nuclei cells detected in outcome of said assays are respectively reported.

On the basis of the above results the following considerations can be made.

Assays results reported on lines 3 and 4 %S show a low percentage of normal cells entering in S phase (23%), in contrast to an high percentage (88%) of the transformed ones after same treatment (see column 4).

These data correspond with the normal/tumor %S ratios following a treatment with CD or the sole vector, which are in fact 1:4 (23:97) and 1:1,1 (88:99) respectively, and confirmed by nucleation-related data reported in table columns 4 to 7. For normal and tumor cells in fact 98% and 97% respectively of mononucleate cells after control assays (see lines 1 and 2), versus 67% and 35% respectively of binucleate cells and 6% and 47% respectively of three-tetranucleate or even with >4 nuclei, after CD treatment can be observed.

A selective inhibition of cytodieresis and subsequent DNA replication of normal cells with respect to the tumor ones by CD is therefore thereby fully demonstrated.

The above mentioned assays however, demonstrate a further important feature of CD action on the system: reversibility.

Following normal cell treatment reported on line 5, which has been carried out for a time equal to that of the treatment reported in line 3 (CD administration from T0 to T48, rest from T48 to T72, BrdU uptake measuring from T72 to T96) it has been in fact finally observed that as much as 92% of said cells is again mononucleate (vs. 98% of the control cells). Moreover, as much as 96% of the plated cells prove still capable of uptaking the BrdU, vs. 97% of control cells. Scientific validity of this conclusion is strengthened by nucleation-related data and BrdU uptake-related data, and by the measured percentage of cells entering phase S after treatment.

The reversibility of the CD action on normal cells, according to these data is therefore very high.

### Example 6: Cytochalasin D lack of interference with 3FT toxic effects on tumor cells.

In order to verify any possible interference between CD and 3FT actions on tumor cells, a series of colony forming assays has been carried out on C3H 10T1/2(*v-src*/*v-myc*).

The cells have been therefore plated on 60mm plates at a 5×10² cell/60mm density, at the culture conditions described in example 3, and the initial time of 12 hours have been subjected to the following treatments.

In a first assay series, 400 nM CD from T0 to T84, along with 30 µM 3FT from T24 to T72 has been administered for verifying possible interference in CD/3FT combined use.

In a second assay series, 3 µM CB from T0 to T84, along with 30 µM 3FT from T24 to T72, has been administered for verifying possible interference in CB/3FT combined use.

In a third assay series, 400 nM CD from T0 to T84, 3, 3 µM CB from T0 to T84, and 30 µM 3FT from T24 to T72, have been administered as controls of the CD/3FT and CB/3FT combined uses.

In a fourth assay series, 0,11% DMSO and H₂O from T0 to T84 have been administered as system control for setting standard deviation.

After the above administrations, cells were treated as described in example 3. The relevant results are reported in the following table 6.

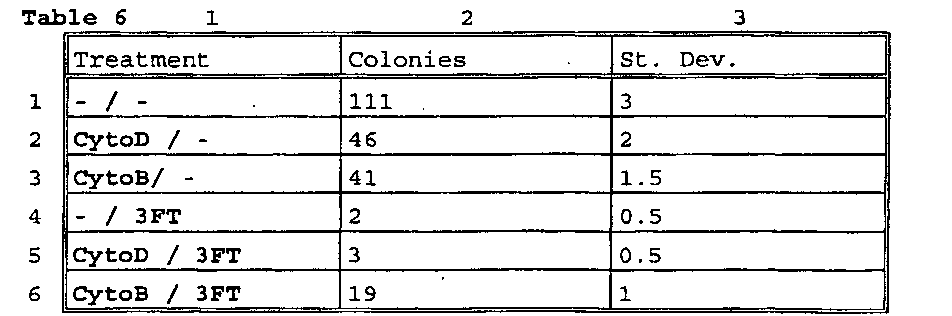

In lines 5, and 6 results of the first and second assay series are respectively reported. In lines 2, 3 and 4 results of the third assay series (inner control assays) are reported. In line 1 are reported the results of the sixth assay series (control assay).

In columns 1, 2 and 3, results of the different treatments carried out on C3H 10T1/2 *v-src*/*v-myc* cells, colony number resulting from each treatment, and standard deviation are respectively reported.

The above results demonstrate the absence of interference with 3FT-dependent killing of tumor cells by CD versus an heavy interference by CB therewith.

After the combined CD/3FT treatment reported in line 5 in fact, 3 colonies versus the 41 of the inner control (line 2) and the 111 of the system control (line 1), have been in fact observed, which represents a nearly total eradication.

After combined CB/3FT treatment of line 6 instead, 19 colonies versus the 46 ones of the inner control and the 111 ones of the system control have been observed, which represents an only partial eradication.

In this way the suitability of cytochalasin B for a complete eradication of tumor cells has been excluded.

These results confirm the inclusion of the cytochalasin D in class A, and the exclusion of cytochalasin B therefrom.

### Example 7: Inhibition of cytodieresis and subsequent DNA replication in normal cells by compounds other than cytochalasin D and reversibility thereof

In order to verify capability of compounds other than cytochalasin D of inhibiting cytodieresis on normal cells, the series of colony-forming assay described in example 5 has been carried out on C3H10T1/2, C3H10T1/2(v-src/v-myc) or on MEF cells with some compounds potentially CD analogues.

In particular the first assay series of example 5 has been carried out with 650 nM Latrunculin B, 700 nM Jasplakinolide, and 100 nM condramide B instead of CD. The second assay series of example 5 has been carried out with 16 µM 13,14,21,22-tetrahydrocytochalasin B, 2 µM 21,22-dihydrocytochalasin B, 1 µM deoxyphomin, 4 µM Cytochalasin F (CF) 8 µM Cytochalasin V (CV) , and 0,5 µM CD as a control on MEF cells.

The relevant results are reported in the following table 7.

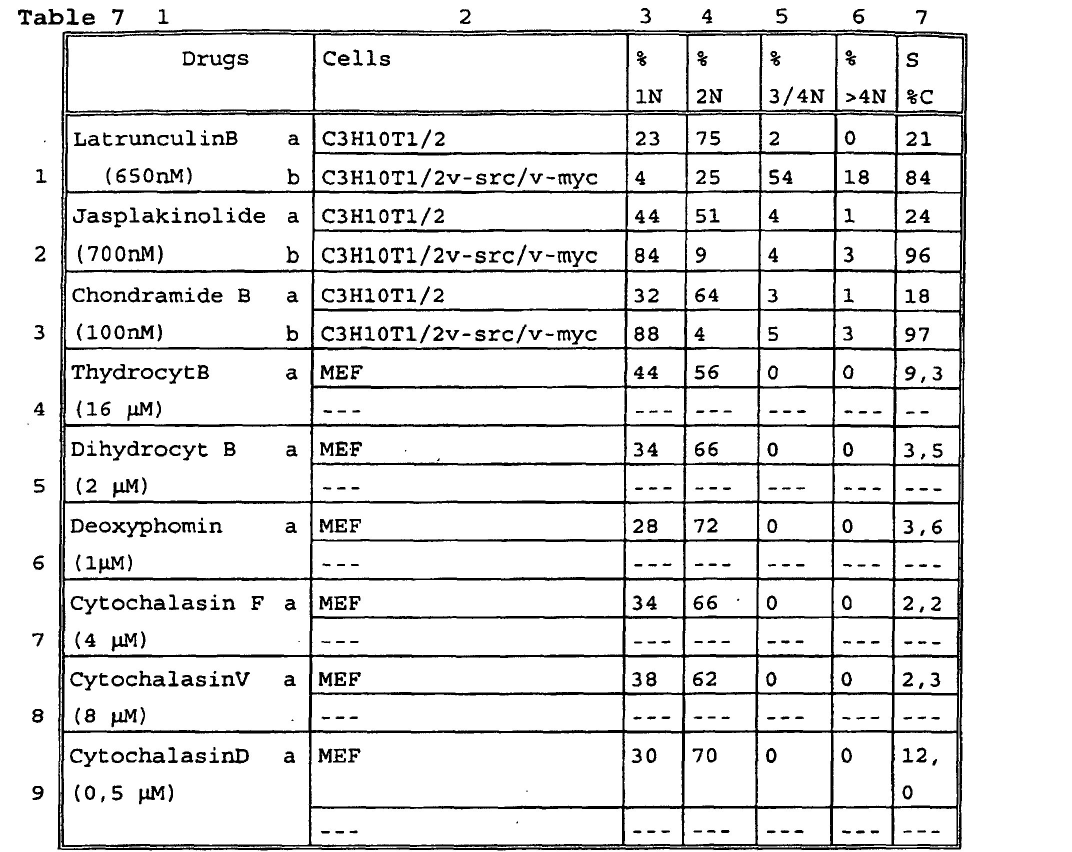

In lines 1 2, and 3, results of first example 5 assay series as carried out administering respectively Latrunculin B, Jasplakinolide, and Chondramide B are reported. In lines 4 to 9, results of the example 5 second assay series as carried out on MEF cells administering 13,14,21,22-tetrahydrocytochalasin B, 21,22-dihydrocytochalasin B, deoxyphomin, Cytochalasin F (CF), Cytochalasin V (CV), and CD as a control, are reported.

In columns 3, 4, 5 and 6 percentages of mononucleate, binucleate, tri-tetranucleate, and >4 nuclei cells detected in outcome of said assays are respectively reported. In column 7 %S values with respect to control are reported. In columns 1 and 2 the administered drugs and the cells assayed are respectively reported.

The above results demonstrate that such compounds are fully capable of interfering with the formation of the contractile ring of the normal cells, the majority thereof remaining at the binucleate stage (see column 4 sublines a).

On transformed cells, instead, all the three assayed compounds (Latrunculin B, Jasplakinolide and Chondramide B) although capable of interfering with the polymerization of the actin cytoskeleton, have an unstable effect to such an extent that cytodieresis inhibition is not evident (see columns 3-6 sublines b). However as these compounds do not interfere with proliferation in tumor cells as widely shown in column 7, they are in this respect suitable for a combined use with 3FT or an analogue thereof.

In any case, in outcome of the above mentioned treatment %S phases and % mononucleate cells have been also verified and are reported in the following table 8, wherein some results of the first assay series described in example 6 carried out on C3H10T1/2 only administering the above compounds are reported in term of % with respect to control).

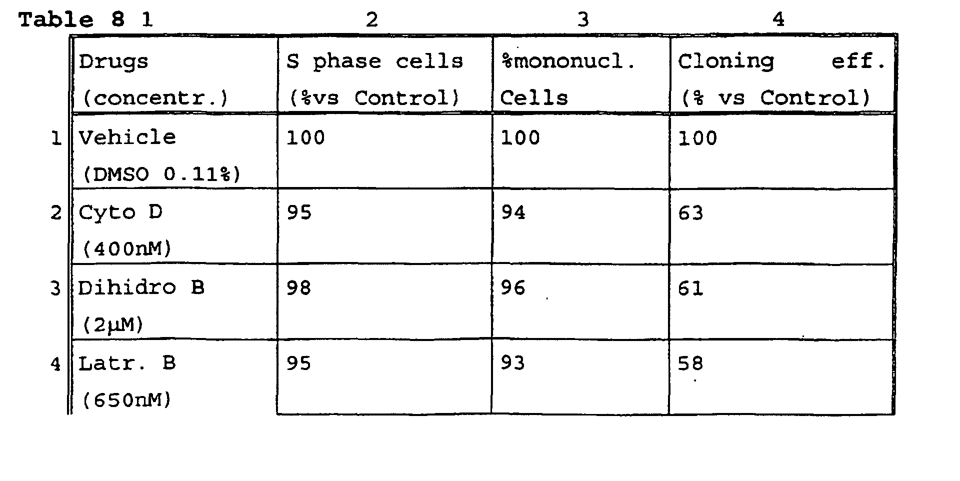

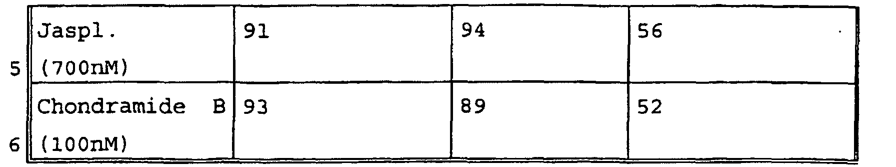

In lines 2 to 6, results of the example 6 first assay series carried out administering Cytochalasin D Dihydrocytochalasin B, Latrunculin B, Jasplakinolide and Chondramide B respectively. In line 1 results of the example 6 control assay carried out administering DMSO only are reported.

In column 1 there are reported the administered drugs and the concentration thereof. In column 2 to 3 there are reported percentage of cells synthesizing DNA after T0-T48 drug administrations and the T0-T96 BrdU uptake, mononucleate cell percentage measured at T96, following the T0-T48 drug administrations and the T0-T96 BrdU uptake with respect to control, and colony percentage measured at the 12th day after T0-T72 drug administrations, followed by drug removal and fresh medium addition from the 4th to the 12th day.

The example 6 first assay series has been in particular carried out for verifying reversibility of each assayed compounds and the cloning efficiency of the cells after the relevant treatment.

The relevant results shown in the table are in particular the ones as referred to administration of 650 nM Latrunculin B, 700 nM Jasplakinolide, 100 nM Condramide B, 2 µM Dihydrocytochalasin B and 400 nM Cytochalasin D as a control. In view of said observed results reversibility for the assayed compound and the consequent suitability in this respect for the 3FT (or equivalent) combined use, have been demonstrated.

The results of the assay series described in example 4 above carried out using dihydrocytochalasin B (referred to as H2CB in the table) and 3FT is reported in the following table 9.

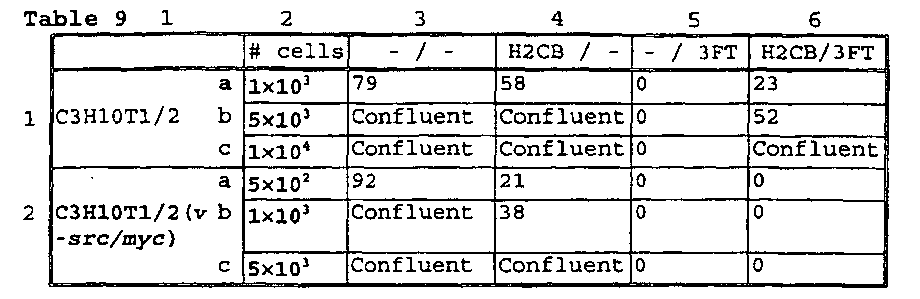

In line 1 results of the first assay series of example 4 as carried out on C3H10T1/2 monoculture, at the clonal densities of 1×10³ (sublane a), 5×10³ (subline b) and 1×10⁴ (subline c) are reported. In line 2 results of the second assay series of example 4 as carried out on C3H 10T1/2*v*-src/*v*-*myc* monoculture at the clonal densities of 5×10² (subline a), 1×10³ (subline b) and 5×10³ (subline c), are reported.

In columns 2, 3, 4 and 5 results of treatment with respectively the sole vehicles (DMSO for CD and H2O for 3FT), the sole CD, the sole 3FT, and CD/3FT, are reported. In column 1 are instead reported the cell assayed and the relevant densities.

### Example 8: Non-interference of compounds other than CD with 3FT uptake by tumor cells

In order to verify a possible interference on 3FT uptake by tumor cells, the compounds mentioned in example 7 have been assayed on C3H10T1/2*v-src*/*v-myc* cells, measuring the uptake of tritiated thymidine. It is well known in fact that the uptake mechanism of the latter and that of 3FT (and generally of antimetabolites) are the same (6).

Cells have been plated in a 24-well plate, with a 5x10⁴ cells/well and the assays have been carried out under standard culture conditions, as described in example 1.

After the initial time of 12 hours c.ca (see above example 1), the following assays have been carried out.

In a first assay 3000 nM CB for 24 h, in a second assay 400 nM CD for 24 h, in a third assays 650 nM Latrunculin B for 24 h, in a fourth assay 700 nM Jasplakinolide for 24 h and in a fifth assay 100 nM Condramide for 24 h, have been administered on the C3H10T1/2*v-src*/*v-myc*.

After said administrations, a 4µCi/ml tritiated Thymidine uptake has been carried out for 5 h for each assay, the above mentioned compounds being still present. Cells have been therefore washed 2x with PBS, extracted with 5% cold trichloroacetic acid and subsequently NaOH-lysed.

The various samples once additioned with scintillation liquid, have been analyzed in a β-counter. The emission values, normalized for the amount of proteins in each sample (quantified by Lowry method) are expressed in count values for min/microgram protein in column 3. As controls there have been considered:
- a first C3H10T1/2*v-src*/*v-myc* cell culture let free to proliferate for the overall assay (12 h + 24 h + 5 h) the radioactivity thereof being determined at the β-counter.
- a second C3H10T1/2*v*-src-myc cell culture let free to proliferate for the initial 12 h and for the pharmacological treatment assay (12 h + 24 h), then subjected to the uptake of 4µCi/ml tritiated thymidine for 5 h and subsequently subjected to uptake determination at the β-counter.

The relevant results are reported in the following table 10.

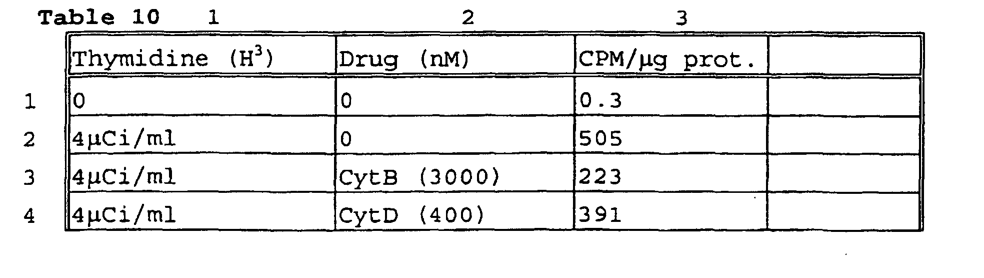

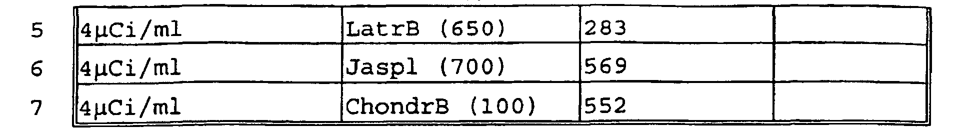

In lines 3 to 7 results of first, second, third, fourth and fifth assays are respectively reported. In lines 1 and 2, results of the first and second control assays are respectively reported.

In columns 1, 2 and 3, the amount of administered Thymidine (H³), the administered drugs administered and concentrations thereof, and emission values in cpm/µg, are respectively reported. The above result analysis confirm that cytochalasin B inhibits nucleoside and therefore antimetabolite transport (223 cpm/µg versus 505 cpm/µg), whereas cytochalasin D substantially does not (391 cpm/µg versus 505 cpm/µg), in compliance with example 6 assays results (see above). Concerning the other assayed compounds, Jasplakinolide and chondramide do not show inhibition of nucleoside transport, with a value of 559 cpm/µg and 562 cpm/µg, respectively, versus the 505 cpm/µg of the control, whereas the Latrunculin B, shows a partial inhibition with a value of 283 cpm/µg.

Such compounds are therefore suitable in the 3FT combined use. The result of these assays and the ones described in example 7 above, have been further in fact confirmed by subjecting all such compounds to the assays described in example 2 and 3 and the positive outcome thereof.

### Example 9: Proved cytotoxicity on proliferating cells and absence of cytotoxicity on arrested normal cell of class B candidate compounds other than 3FT

In order to verify cytotoxicity on proliferating cells of compounds other than 3FT having however an analogous action thereto, the assay described in example 3 above have been carried out using 2-chlorine-deoxyadenosine (2ClAd) and cytarabine (Ara C) instead of 3FT.

The focus-forming assay conditions are those described in example 3 for the first second and third assay series, except that instead of 3FT, 80 µM 2ClAd and 40µM Ara C have been administered. Such concentrations are those predetermined to be the minimum ones for killing at least 90% of the proliferating cells within the time of the combined treatment, as function of the cell cycle of the tumor cells used (48 h in this case).

The relevant results are reported in the following tables 11 A (2ClAd) and B (Ara C)

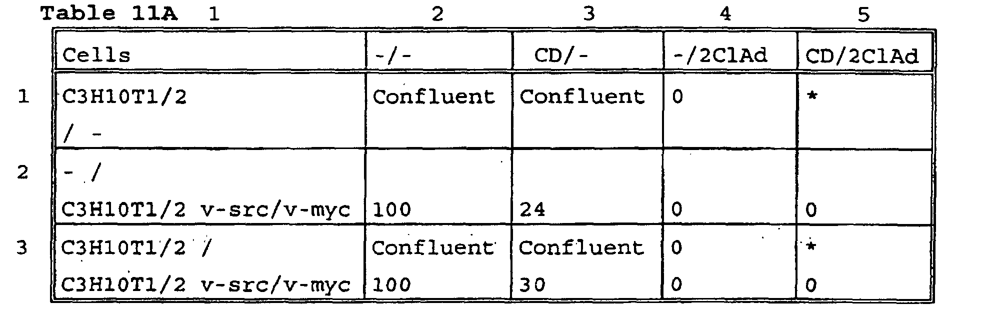

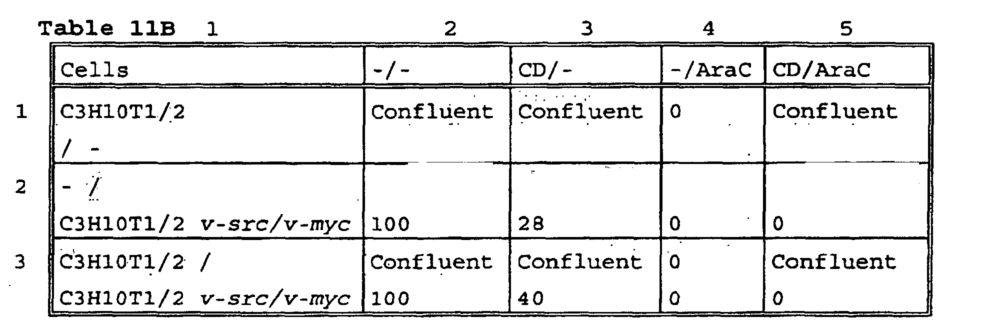

In table 11 A (lines 1, 2 and 3) there are respectively reported the results of the example 3 first, second and third assay series, as carried out administering 2ClAd instead of 3FT.

In table 11 B (lines 1, 2 and 3) there are respectively reported the results of the example 3 first, second and third assay series, as carried out administering Ara C instead of 3FT.

In column 2, 3, 4 and 5 of both tables results of treatment with respectively the sole vehicles (DMSO for CD and H2O for 3FT), the sole CD, the sole 3FT, and CD/3FT, are reported. In column 1 there are reported the cell assayed.

Both in the assay series carried out administering AraC and the ones carried out administering 2ClAd, a drastic diminution, or even the absence of tumor cells colonies and transformation foci, and also reforming of the normal cell monolayer (see column 4 of both panels) have been observed.

However, while the monolayer of cells subjected to CD/AraC combined treatment reforms normally, the requested time for normal cell monolayer reforming after the CD/2ClAd combined treatment doubles that of the two other assayed compounds (from the 5 days of the assay carried out with Ara C and 3FT to the 10/12 days of the assay carried out with 2ClAd). Moreover, the monolayer saturation density (number of cells per surface unit) reformed after the 2ClAd treatment is markedly lower with respect to the other cases (of about 1/3), and the cells thereof appear quite senescent.

These data related to the proliferation restart phase and to the regrowth of normal cells at the end of the whole treatment, demonstrate a selective cytotoxic effect of AraC on tumor cells, as opposed to a certain toxic effect of the 2ClAd, not only on tumor cells but also on the normal ones after the combined treatment. Such a datum has been therefore verified with the assays reported in the following examples 10 and 11.

### Example 10: Absence of cytotoxicity on growth-arrested cells by 3FT and test of candidate class B compounds other than 3FT.

A series of DNA synthesis reactivation assays following treatments with the compounds described in the above example 9 (Ara C and 2ClAd) and with other compounds having an action analogous to 3FT (6-thioguanine (6ThG) and doxorubicine), have been carried out on C3H10T1/2 cells reversibly arrested in G0/G1 by growth factor deprivation.

The choice of carrying out the assays on cells arrested in the G0/G1 phase by growth factor deprivation rather than on cells treated with cytochalasin D or analogues thereof was made for ease of assaying. Data therefrom are considered as relevant to the present discussion, since normal cells treated with cytochalasin D have been observed to arrest at a binucleate stage in the G0/G1 phase of the cell cycle (19).

The cells have been plated at a 10⁵ density per each 35mm plate and the culture conditions are the standard ones defined in example 1.

After the initial time of 12 hours c.ca (see above example 1), the cells have been transferred to a low growth factor medium (1% FCS-additioned DMEM) to arrest said proliferation. 48 h later, (after having determined a cell condition in all analogous to the situation at T24 according to the treatment scheme reported in Fig. 1 and described in examples 3 and 4) the following pharmacological treatments have been carried out.

In a first assay 40µM 3FT for 48 h, in a second assay Ara C 40µM for 48 h, in a third assay 80µM 2ClAd for 48 h, in a fourth assay 100µM 6 thioguanine (6Thg) for 48 h, in a fifth assay 1µg/ml Doxorubicine (Doxo) for 48 h have been administered on the G0/G1 arrested C3H10T1/2 cells.

The above concentrations are the minimum for killing proliferating cells calculated on the basis of cell cycle duration.

In a sixth assay 1µM Ethidium Bromide (EtBr) for 48 h, and in a seventh assay 20 mM BrdU for 48 h have been administered as a positive and negative control respectively.

Treatment time was equal to that of the example 3 third assay series above, carried out however on the C3H10T1/2 arrested cells.

After release from drug treatment, cells were washed 2x and left in low serum for other 12 h, to allow washing of residual drugs under non-proliferating conditions.

After the 24 h period in low serum, proliferation medium (DMEM +10% FCS), supplemented with 20µM BrdU in order to mark the DNA-synthesizing cells, has been additioned to the cell culture. After a 24-h stimulation, the cells have been fixed and processed as reported in Grossi et al. 1991 (18). The percentage of DNA-synthesizing cells in the 24-h labeling period has been detected by immunofluorescence, as reported in Grossi et al. 1991 (18).

Both the total cell number, which is a direct indication of the cytotoxic action of the compounds (expressed as percentage with respect to the untreated sample), and the number of cells maintaining intact proliferation potential (i.e., cells capable of replicating their DNA), have been detected. In this way the untreated controls have always been considered. The relevant results are reported in the following table 12.

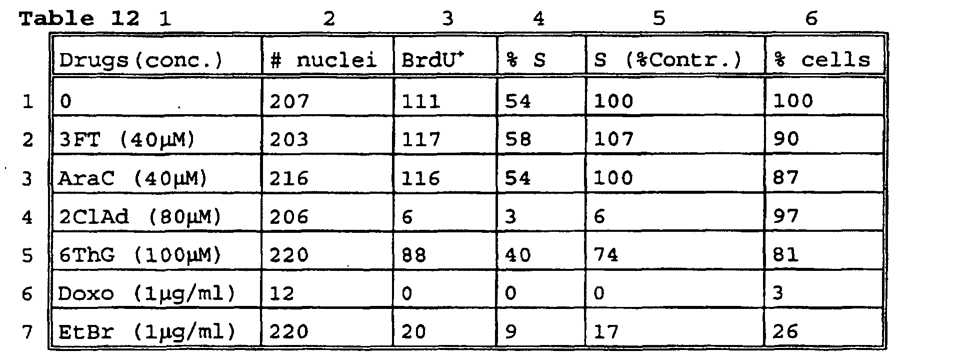

In lines 2 to 6, results of the first, second, third, fourth and fifth assay series are reported. In lines 7 and 1, results of sixth and seventh assays (positive and negative control) are respectively reported.

In columns 2 to 4, the total number of observed nuclei, the number of nuclei having uptaken BrdU after treatment, and percentage of cells entering S phase extrapolated on the basis of the data reported in columns 2 and 3 are respectively reported. In column 5 percentage of cells entering S phase referred to non treated control cells of line 1 extrapolated on the basis of the data reported in columns 4, are reported for each assay. In column 6 percentage of cells eventually still present on the plate after the treatment (as compared to the untreated control) are reported for each assay. In column 1 the different administered drugs and the concentrations used are reported.

On the basis of the above reported surviving cell number and number of cells proved also capable of reentering the cell cycle in outcome to these assays, results of example 7 assay as referred to 2ClAd have been confirmed. Following 2ClAd administration, in fact, although a high percentage of residual adherent cells (97%) has been observed, few thereamong actually maintained their proliferation potential (a mere 3%- 6% when compared to the untreated control). As a consequence it has been confirmed that DNA synthesis was almost totally inhibited, regardless of the observation related to the sole cell presence.

Concerning the other assayed drugs (3FT, Ara C, 6ThG), instead, they proved non-toxic with respect to cells arrested at the intermitotic phase, whereas the doxorubicine, although considered in the art as a potential drug to be used for tumor cell eradication, proved to be too toxic on normal cells growth-arrested in interphase for allowing maintenance of proliferative potential. Accordingly while 3FT, Ara C, 6ThG have been demonstrated to be suitable in combination with CD and analogues thereof, for a complete and selective eradication of tumor cells, both 2ClAd and doxorubicine did not.

### Example 11: Cytotoxicity quantification of 3FT and analogues by increasing the relevant concentrations

In order to verify whether, and to what extent concentrations of the 3FT and analogues thereof used in the assay series reported in example 10 (wherein the minimum ones allowing to obtain a cytotoxic effect on proliferating tumor cells have been used) could be varied for enhancing the eradication effectiveness per time unit, the assay series reported in example 10 with increasing concentrations of said compounds has been carried out on C3H10T1/2 cells reversibly arrested in the G0/G1b phase by growth factor deprivation.

The culture and assay conditions are those described in example 10, except for the pharmacological treatment, which in the present assay series consists of the following.

In a first assay series, Ara C 50 µM, 100µM, 200 µM, 400 µM and 800µM, and as a control Cytidine 50 µM, 100µM, 200 µM, 400 µM and 800µM, have been administered for 48 h to the above mentioned arrested cells.

In a second assay series 3FT 50 µM, 100µM, 200 µM, 400 µM and 800µM, and as a control Thymidine 50 µM, 100µM, 200 µM, 400 µM and 800µM have been administered for 48 h to the above mentioned cells.

In particular assays carried out with Cytidine for the Ara C and Thymidine for the 3FT have been carried out in order to avoid any possible toxicity data distortion deriving from the use of increased nucleoside concentrations.

Three further control assays wherein 20 µM BrdU, 80µM 2ClAd and 1µg/ml Doxorubicine, have been administered for 48 h to the above mentioned cells, have been however carried out.

The highest compound concentrations in all these assays were 10-20 fold increased with respect to the minimum dose required for the cell eradication in the combined use with CD or analogue thereof. This has allowed to verify whether eradication effectiveness could be improved (with regard, above all, to the *in vivo* or *ex vivo* treatment) by increasing the chemotherapeutic drug concentration, overcoming dosage limitation due to the toxicity thereof.

The relevant results are reported on the following table 13.

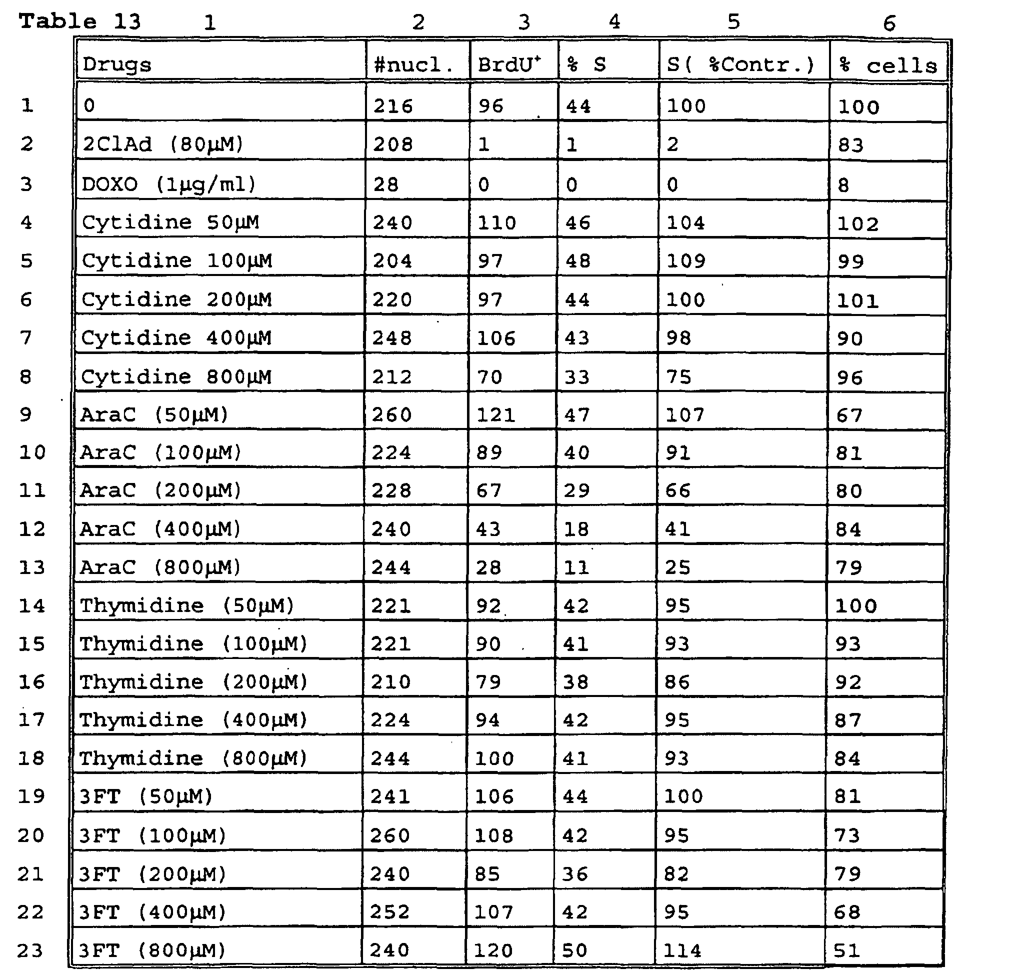

In lines 9 to 13, results of the first assay series are reported. In lines 4 to 8 results of the relevant control assay series are reported. In lines 19 to 23, results of the second assay series are reported. In lines 14 to 18 results of the relevant control assay series are reported. In lines 1 to 3 the results of the three further control assays carried out administering respectively BrdU, 2ClAd and Doxorubicine, are reported.

In columns 2 to 4, the total number of observed nuclei, the relative number of nuclei having uptaken BrdU after treatment, and percentage of cells entering S phase calculated on the basis of the data reported in columns 2 and 3 are respectively reported. In column 5 percentage of cells entering S phase referred to non treated control cells of line 1 calculated on the basis of the data reported in columns 4, are reported for each assay. In column 6 percentage of cells eventually still adherent after the treatment (as compared to the untreated control) are reported for each assay. In column 1 the different administered drugs and the concentrations used are reported.

The above results prove that the trifluorothymidine starts to be toxic at 400-800 µM, since the percentage of cells that enter the S phase is unaltered with respect to the other assays but the number of observed cells is approximately halved (see lines 22-23), whereas the Ara-C starts to be toxic at 200-400 µM, since in this case the number of observed cells remains the same, but the percentage of cells capable of entering S phase decreases, (see lines 11-12).

On the basis of these results, even and above all as compared to those related to the two control compounds 2ClAd and Doxo, resulting in 0 growth, it can be stated that by virtue of the combined use with cytochalasin D (or an analogue thereof) the concentration of the above mentioned compound (or an analog thereof) can be increased up to 10-fold, since a certain toxicity thereof is anyhow negligible. Concerning the optimum dosages, the 3FT appears to be well-tolerated up to 400/800µM concentrations. Instead, AraC appears to begin to be toxic at 400µM dosage.

### Example 12: Confirming method applicability to human tumor and normal cell lines

All the assays above described, have been carried out in a system specifically designed to reproduce pathological situations in human beings in vivo. In order to confirm the full applicability of the results obtained from the C3H10T1/2 cells to human cells, an assay series has been carried out on cultured cells coming from glioblastomas.

Culture and experimental conditions are identical to those reported in example 1, except in that the cells used were Lipari, T97 and U973 glioblastomas and that nucleation state data were observed after the following treatments.

In a first assay series, 400 nM cytochalasin D for 48 h (T0-T48), along with BrdU (T24-48) in the final 24 h for controlling the percentage of cells capable of entering S phase, has been administered to Lipari, T97 and U973 cells.

In a second assay series, 650 nM Latrunculin B for 48 h (T0-T48) along with BrdU (T24-48) in the final 24 h for controlling the percentage of cells capable of entering S phase, has been administered to Lipari (Line 11), T97 (Line 12) and U973 (Line 13) cells.

In a third assay series, 700 nM Jasplakinolide for 48 h (T0-T48), along with BrdU (T24-48) in the final 24 h for controlling the percentage of cells capable of entering S phase, has been administered to Lipari (Line 14), T97 (Line 15) and U973 (Line 16) cells.

In a fourth assay series, 100 nM Chondramide B for 48 h (T0-T48), along with BrdU (T24-48) in the final 24 h for controlling the percentage of cells capable of entering S phase, has been administered to Lipari (Line 17), T97 (Line 18) and U973 (Line 19) cells.

In a fifth assay series, 400 nM cytochalasin D for 48 h (T0-T48), along with BrdU (T24-48) in the final 24 h for controlling the percentage of cells capable of entering S phase, has been administered to lung (9) and dermis (10) human fibroblasts.

In control assays, 20 µM BrdU only has been administered for 48 h to Lipari (Line 1), T97 (Line 2) and U973 (Line 3) cell lines, lung (4) and dermal (5) fibroblasts.

In particular first there have been carried out the above mentioned first to fourth assays on Lipari, T97 and U973. Secondly, following the positive outcome, as a further confirmation the fifth assay on lung fibroblasts (the same used for example 2 assays), and dermis fibroblasts, have been carried out administering CD only. The relevant results are reported in the following table 14.

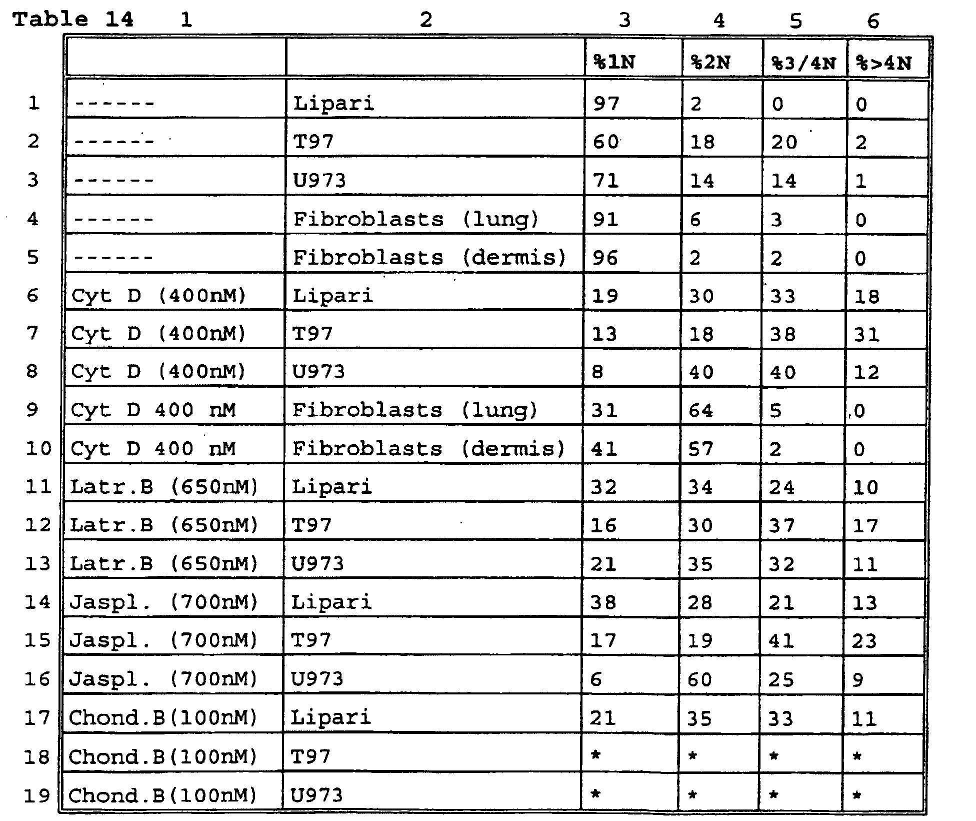

In lines 6 to 8, results of the first assay series as carried out on Lipari, T97 and U973 respectively are reported. In lines 11 to 13 results of the second assay series as carried out on Lipari, T97 and U973 respectively are reported. In lines 14 to 16 results of the third assay series as carried out on Lipari, T97 and U973 respectively are reported. In lines 17 to 19 results of the fourth assay series. as carried out on Lipari, T97 and U973 respectively are reported. In lines 9 and 10 results of the fifth assay series as carried out on Lipari, T97 and U973 respectively are reported. In lines 1 to 5 results of the control assays, as carried out respectively on Lipari, T97, U973, lung and dermis fibroblasts are reported.

In columns 3, 4, 5 and 6 percentages of mononucleate, binucleate, tri-tetranucleate, and >4 nuclei cells detected in outcome of said assays are respectively reported. In column 1 and 2 are instead reported the administered drug and the assayed cell assayed are reported.

Results of all these assay series highlight multinucleation of all the human cells treated with the above compounds (see results related to the percentage of tri-tetra-, or >4- nucleate cells reported in columns 5 and 6, respectively).

In particular, chondramide B shows a particular effective action, as not only it induces multinucleation of tumor cells, but it is even capable of causing their detachment.

Even if the data are not herein reported, the reversibility of said compounds action has also been proved in such human cells as reported for murine cells (see Ex. 5, 7). This confirms the applicability of the combined use according to the present invention also to the human system, and the full extensibility to the human system of the results obtained on the model system, the latter being anyhow validated thereby.

### GLOSSARY

- The term "tumor cells" as used here relates:
   *in vivo,* to cells constituting the various tumor forms;
   *in vitro*, to *in vitro* transformed cells (i.e., having become tumorigenic either spontaneously or by an oncogen expression) or deriving from tumor explants, however not meeting the requirements indicated in the definition of the normal *in vitro* cells (1-3).
- The term "normal cells" as used here relates:
   *in vivo*, to any body cell not in a transformed or tumoral state;
   *in vitro*, to primary or culture-stabilized cells, having maintained the characteristics identified as normal in the state of the art, i.e.:
      - dependence on growth factors for the proliferation;
      - inability to form tumors when injected in immunodeficient mice;
      - inability to form transformation foci in the assays provided for the purpose; and
      - dependence on anchorage for growth (only for those cells usually growing adherent).
- With the locution "functional p53 pathway", as here used, it is referred to the complex of genes whose products, acting upstream and downstream of p53 gene or gene product, are required for the proper functioning of p53 in regulating cell cycle and genome stability.

### References

1. De vita VT, Helman S, Rosenberg SA - CANCER 5^{th} edition; Lippincott-Raven.
2. Natori S - Toxic cytochalasins. *Mycotoxins* 1978 May 24 pp.;: 559-81 Kunze B, Jansen R,
3. Bray D - Cytochalasin action Nature 1979 Dec 13;282(5740):671
4. Smith GF, Ridler MA, Faunch JA - Action of cytochalasin B on cultured human lymphocytes. *Nature* 1967 Dec 16;216(120):1134-5
5. Kelly F, Sambrook J - Differential effect of cytochalasin B on normal and transformed mouse cells. *Nat New Biol* 1973 Apr 18;242 (120) :217-9
6. Plagemann PG, Estensen RD - Cytochalasin B. VI. Competitive inhibition of nucleoside transport by cultured Novikoff rat hepatoma cells. *J Cell Biol* 1972 Oct;55(1):179-85
7. Zylka JM, Plagemann PG - Purine and pyrimidine transport by cultured Novikoff cells. Specificities and mechanism of transport and relationship to phosphoribosylation. *J Biol Chem* 1975 Aug 10;250 (15) :5756-67
8. Tanaka K, Kuwano M, Endo H - Effect of cytochalasin-B on the transport of 5-fluorouracil in cultured mammalian cells. *Gann* 1975 Jun;66(3):331-4
9. Scott VR, Boehme R, Matthews TR- New class of antifungal agents: jasplakinolide, a cyclodepsipeptide from the marine sponge, Jaspis species. *Antimicrob Agents Chemother* 1988 Aug;32(8):1154-7
10.Sasse F, Hofle G, Reichenbach H - Chondramides A approximately D, new antifungal and cytostatic depsipeptides from Chondromyces crocatus (myxobacteria). Production, physico-chemical and biological properties. J. Antibiot (Tokio) 1995 48 (11) : 1262-6
11.Udagawa Taturo, D'Amato RJ, Shah JH - Cytochalasin and Isoindolinone derivatives as inhibitors of angiogenesis. World patent WO98/41205 (18.03.98)
12.Spector I, Shochet NR, Kashman Y, Groweiss A - Latrunculins: novel marine toxins that disrupt microfilament organization in cultured cells. *Science* 1983 Feb 4;219(4584):493-5
13.McClatchey AI, Jacks T Tumor suppressor mutations in mice: the next generation. *Curr Opin Genet Dev* 1998 Jun;8(3):304-10
14.Prives C, Hall PA - The p53 pathway. *J Pathol* 1999 jan; 187(1): 112-26
15.Waldman T, Lengauer C, Kinzler KW, Vogelstein B - Uncoupling of S phase and mitosis induced by anticancer agents in cells lacking p21. *Nature* 1996 381: 643-644
16.O'Neill FJ, Miller TH, Hoen J, Stradley B, Devlahovich V - Differential response to cytochalasin B among cells transformed by DNA and RNA tumor viruses. *J Natl Cancer Inst* 1975 Oct;55(4) :951-5
17.Bignami M, Rosa S, La Rocca SA, Falcone G, Tatò F - Differential influence of adjacent normal cells on the proliferation of mammalian cells transformed by the viral oncogenes myc, ras and src. *Oncogene* 1988 May;2(5):509-14
18.Grossi M, Calconi A, Tatò F - v-jun oncogene prevents terminal differentiation and suppresses muscle-specific gene expression in ASV-17-infected muscle cells. *Oncogene* 1991 Oct;(10): 1767-73
19.Zanetti A, Grossi M, Tatò F - Manuscript in preparation

## Claims

1. A method for protecting proliferating normal cells in an *in vitro* culture from the eradicative action of a chemotherapeutic compound (class B compound), the culture comprising said proliferating normal cells and tumor cells having an inactive p53 pathway,
said method comprising administering to said culture the chemotherapeutic compound in combination with a protective compound (class A compound),
the chemotherapeutic compound having the capability of
- exerting a cytotoxic action toward actively proliferating cells,
- not affecting survival and proliferative potential of interphase cells, the protective compound having the capability of
- reversibly inhibiting cytodieresis of normal cells,
- not inhibiting the biological action of said chemotherapeutic compound and,
wherein a pre-treatment with said protective compound is carried out before the combined treatment with class A and class B compounds and the administration of said protective compound results in the protection of at least part of said proliferating normal cells.

2. The method according to claim 1, wherein the pre-treatment with the protective compound results in the arrest at interphase of at least part of said normal cells.

3. The method according to claim 2, wherein the pre-treatment is carried out for a time greater than or equal to the cell cycle duration of said proliferating normal cell.

4. The method according to claims 1 or 3, wherein, after the combined treatment, a post treatment is carried out including interrupting the administration of said class B compound and washing said class B compound off the culture while maintaining the administration of said class A compound.

5. The method according to any of claims 1 to 4, wherein the combined treatment is carried out for a time greater than or equal to the cell cycle duration of said tumor cells'having an inactivated p53 pathway.

6. The method according to claims 4 or 5, wherein said washing step is carried out for a time greater than or equal to 3 hours.

7. The method according to any of claims 1 to 4 wherein said. combined treatment, pre-treatment and/or post-treatment is repeated twice or more.

8. A method according to any one of claims 1 to 7, wherein the protective compound is selected from the group consisting of cytochalasins, jasplakinolides, chondramides, isoindolinones, and latrunculines, with the exclusion of cytochalasin B.

9. A method according to claim, 8, wherein said protective compound is selected from the group consisting of the cytochalasin D, dihydrocytochalasin B, jasplakinolide, chondramide B and latrunculin B.

10. A method according to any one of claims 1 to 9, wherein the chemotherapeutic compound is selected from the group consisting of folate inhibitors, nucleoside analogues, nucleotide synthesis inhibitors, vinca alkaloids, taxanes, colchicine derivatives, podophillotoxin derivatives, and topoisomerase inhibitors.

11. A method according to claim 10, wherein the chemotherapeutic compound is selected from the group consisting of trifluorothymidine, cytarabine, 6-thioguanine, 6-mercaptopurine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, 9-amino-S(20)-camptothecine.

12. The method according to any one of claims 1 to 11, wherein C3H10T1/2 cells or cells derived therefrom are used as model cells.

13. The method according to any one of claims 1 to 11, wherein C3H10T1/2 cells or cells derived therefrom are used as model cells for identifying a protective compound and/or a chemotherapeutic compound.

14. Use of a protective compound having the capability of
- reversibly inhibiting cytodieresis of normal cells,
- not inhibiting the biological action of a chemotherapeutic compound, which chemotherapeutic compound has the capability of
- exerting a cytotoxic action toward actively proliferating cells, but
- not affecting survival and proliferative potential of interphase cells,
for the preparation of a medicament for protecting normal cells from the eradicative action of said chemotherapeutic compound in a treatment of a tumor form having an inactivated p53 pathway.

15. Use according to claim 14, wherein the combined treatment with the protective compound and the chemotherapeutic compound follows a pre-treatment with the protective compound alone.

16. The use according to claim 15, wherein the pre-treatment is carried out for a time greater than or equal to the cell cycle duration of said proliferating normal cell.

17. Use according to any one of claims 14 to 16, wherein said tumor form is a tumor form having a low proliferating potential.

18. Use according to any one of claims 14 17, wherein said tumor form is an hyperproliferative lesion caused by papillomavirus.

19. Use of a protective compound as defined in claim 14 for the preparation of a medicament for protecting normal cells from the eradicative action of a chemotherapeutic compound in a treatment of a pathological infection caused by microorganisms displaying no p53 function.

20. Use of a protective compound as defined in claims 14 for the preparation of a medicament for preventing and treating alopecia associated to a systemic administration with a chemotherapeutic compound in a treatment of cells having an inactivated p53 pathway.

21. Use according to any one of claims 14 to 20 wherein the protective compound is selected from the group consisting of cytochalasins, jasplakinolides, chondramides, isoindolinones, and latrunculines, with the exclusion of cytochalasin B.

22. Use according to claim 21 wherein said protective compound is selected from the group consisting of the cytochalasin D, dihydrocytochalasin B, jasplakinolide, chondramide B and latrunculin B.

23. Use according to any one of claims 14 to 22, wherein the chemotherapeutic compound is selected from the group consisting of folate inhibitors, nucleoside analogues, nucleotide synthesis inhibitors, vinca alkaloids, taxanes, colchicine derivatives, podophillotoxin derivatives, and topoisomerase inhibitors.

24. Use according to claim 23, wherein the chemotherapeutic compound is selected from the group consisting of trifluorothymidine, cytarabine, 6-thioguanine, 6-mercaptopurine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, 9-amino-S(20)-camptothecine

25. A pharmaceutical composition for selectively eradicating cells having inactive p53 pathway comprising therapeutically effective amounts of a protective compound, a chemotherapeutic compound and a pharmaceutically acceptable vehicle, carrier or auxiliary agent, the chemotherapeutic compound having the capability of
- exerting a cytotoxic action toward actively proliferating cells,
- not affecting survival and proliferative potential of interphase cells,
the protective compound having the capability of
- reversibly inhibiting cytodieresis of normal cells,
- not inhibiting the biological action of said chemotherapeutic compound, wherein the release of the chemotherapeutic compound is retarded with respect to the release of the protective compound.

26. Pharmaceutical composition according to claim 25, wherein the chemotherapeutic compound is selected from the group consisting of folate inhibitors, nucleoside analogues, nucleotide synthesis inhibitors, vinca alkaloids, taxanes, colchicine derivatives, podophillotoxin derivatives, and topoisomerase inhibitors.

27. Pharmaceutical composition according to claim 26, wherein the wherein said chemotherapeutic compound is selected from the group consisting of trifluorothymidine, cytarabine, 6-thioguanine, 6-mercaptopurine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, 9-amino-S(20)-camptothecine.

28. Pharmaceutical composition according to claim 25, wherein said protective compound is selected from the group consisting of cytochalasins, jasplakinolides, chondramides, isoindolinones, and latrunculines, with the exclusion of cytochalasin B.

29. A pharmaceutical composition according to claim 28 wherein said protective compound is selected from the group consisting of the cytochalasin D, dihydrocytochalasin B, jasplakinolide, chondramide B and latrunculin B.

30. A kit of parts for selectively eradicating cells having an inactive p53 pathway and selectively protecting proliferating normal cells comprising a protective compound and a chemotherapeutic compound, the chemotherapeutic compound having the capability of
- exerting a cytotoxic action toward actively proliferating cells,
- not affecting survival and proliferative potential of interphase cells,
the protective compound having the capability of
- reversibly inhibiting cytodieresis of normal cells,
- not inhibiting the biological action of said chemotherapeutic compound, the kit being for the sequential administration of the protective compound alone firstly, and then of the association of the protective and chemotherapeutic compounds in an *in vivo* and/or *ex vivo* therapy of a tumor form having an inactive p53 pathway.

31. A kit of parts according to claim 30, wherein said protective compound is selected from the group consisting of cytochalasins, jasplakinolides, chondramides, isoindolinones, and latrunculines, with the exclusion of cytochalasin B.

32. A kit of parts according to claim 30, wherein said protective compound is selected from the group consisting of cytochalasin D, dihydrocytochalasin B, jasplakinolide, chondramide B and latrunculin B.

33. A kit of parts according to claim 30, wherein said chemotherapeutic compound is selected from the group consisting of folate inhibitors, nucleoside analogues, nucleotide synthesis inhibitors, vinca alkaloids, taxanes, colchicine derivatives, podophillotoxin derivatives, and topoisomerase inhibitors.

34. A kit of parts according to claim 33, wherein said chemotherapeutic compound is selected from the group consisting of trifluorothymidine, cytarabine, 6-thioguanine, 6-mercaptopurine, gemcytabine, fludarabine, floxuridine, ftorafur, methotrexate, trimetrexate, raltitrexed, edatrexate, lometrexol, hydroxyurea, vincristine, vinblastine, vinorelbin, vindesine, paclitaxel, docetaxel, irinotecan, topotecan, 9-amino-S(20)-camptothecine.

35. A kit of parts according to any one of claims 30 to 34 wherein said tumor form is a hyperproliferative lesion caused by papillomavirus infection.

36. A kit of parts according to any of claims 30 to 34 in the therapy of a pathological infection associated to a microorganism having no p53 function, wherein the administration of the combined compounds follows the administration of the protective compound alone.

37. A kit of parts according to any of claims 30 to 34 for selectively eradicating cells having an inactive p53 function and selectively protecting proliferating normal cells in the method according to any of claims 1 to 12.

## Patentansprüche

1. Verfahren zum Schützen von proliferierenden normalen Zellen in einer *in vitro*-Kultur vor der eradikativen Wirkung einer chemotherapeutischen Verbindung (Klasse B-Verbindung), wobei die Kultur die proliferierenden normalen Zellen und Tumorzellen mit einem inaktiven p53-Stoffwechselweg umfasst,
wobei das Verfahren das Verabreichen der chemotherapeutischen Verbindung in Kombination mit einer schützenden Verbindung (Klasse A-Verbindung) an die Kultur umfasst,
wobei die chemotherapeutische Verbindung die Fähigkeit hat
- eine cytotoxische Wirkung gegen aktiv proliferierende Zellen auszuüben,
- das Überleben und proliferative Potenzial von Interphasezellen nicht zu beeinträchtigen,
wobei die schützende Verbindung die Fähigkeit hat
- die Zellteilung (cytodieresis) von normalen Zellen reversibel zu hemmen,
- die biologische Wirkung der chemotherapeutischen Verbindung nicht zu hemmen, und
wobei eine Vorbehandlung mit der schützenden Verbindung vor der kombinierten Behandlung mit Klasse A- und Klasse B-Verbindungen durchgeführt wird und die Verabreichung der schützenden Verbindung dazu führt, dass wenigstens ein Teil der proliferierenden normalen Zellen geschützt ist.

2. Verfahren nach Anspruch 1, wobei die Vorbehandlung mit der schützenden Verbindung dazu führt, das wenigstens ein Teil der normalen Zellen in der Interphase angehalten wird.

3. Verfahren nach Anspruch 2, wobei die Vorbehandlung während eines Zeitraums erfolgt, der größer oder gleich der Dauer des Zellzyklus der proliferierenden normalen Zelle ist.

4. Verfahren nach den Ansprüchen 1 oder 3, wobei nach der kombinierten Behandlung eine Nachbehandlung erfolgt, welche das Unterbrechen der Verabreichung der Klasse B-Verbindung und das Abwaschen der Klasse B-Verbindung von der Kultur unter Beibehaltung der Verabreichung der Klasse A-Verbindung umfasst.

5. Verfahren nach einem der Ansprüche 1 bis 4, wobei die kombinierte Behandlung während eines Zeitraums erfolgt, der größer oder gleich der Dauer des Zellzyklus der Tumorzellen mit einem inaktivierten p53-Stoffwechselweg ist.

6. Verfahren nach den Ansprüchen 4 oder 5, wobei der Waschschritt während eines Zeitraums erfolgt, der größer oder gleich 3 Stunden ist.

7. Verfahren nach einem der Ansprüche 1 bis 4, wobei die kombinierte Behandlung, Vorbehandlung und/oder Nachbehandlung zweimal oder öfter wiederholt wird.

8. Verfahren nach einem der Ansprüche 1 bis 7, wobei die schützende Verbindung ausgewählt ist aus der Gruppe bestehend aus Cytochalasinen, Jasplakinoliden, Chondramiden, Isoindolinonen und Latrunculinen, wobei Cytochalasin B ausgeschlossen ist.

9. Verfahren nach Anspruch 8, wobei die schützende Verbindung ausgewählt ist aus der Gruppe bestehend aus dem Cytochalasin D, Dihydrocytochalasin B, Jasplakinolid, Chondramid B und Latrunculin B.

10. Verfahren nach einem der Ansprüche 1 bis 9, wobei die chemotherapeutische Verbindung ausgewählt ist aus der Gruppe bestehend aus Folat-Inhibitoren, Nukleosid-Analoga, Nukleotidsynthese-Inhibitoren, Vinca-Alkaloiden, Taxanen, Colchicin-Derivaten, Podophyllotoxin-Derivaten und Topoisomerase-Inhibitoren.

11. Verfahren nach Anspruch 10, wobei die chemotherapeutische Verbindung ausgewählt ist aus der Gruppe bestehend aus Trifluorthymidin, Cytarabin, 6-Thioguanin, 6-Mercaptopurin, Gemcytabin, Fludarabin, Floxuridin, Ftorafur, Methotrexat, Trimetrexat, Raltitrexed, Edatrexat, Lometrexol, Hydroxyharnstoff, Vincristin, Vinblastin, Vinorelbin, Vindesin, Paclitaxel, Docetaxel, Irinotecan, Topotecan, 9-Amino-S(20)-camptothecin.

12. Verfahren nach einem der Ansprüche 1 bis 11, wobei C3H10T1/2-Zellen oder davon abgeleitete Zellen als Modellzellen verwendet werden.

13. Verfahren nach einem der Ansprüche 1 bis 12, wobei C3H10T1/2-Zellen oder davon abgeleitete Zellen als Modellzellen zum Identifizieren einer schützenden Verbindung und/oder einer chemotherapeutischen Verbindung verwendet werden.

14. Verwendung einer schützenden Verbindung mit der Fähigkeit
- die Zellteilung von normalen Zellen reversibel zu hemmen,
- die biologische Wirkung einer chemotherapeutischen Verbindung nicht zu hemmen, wobei diese chemotherapeutische Verbindung die Fähigkeit hat
- eine cytotoxische Wirkung gegen aktiv proliferierende Zellen auszuüben, aber
- das Überleben und proliferative Potenzial von Interphasezellen nicht zu beeinträchtigen,
für die Herstellung eines Medikaments zum Schützen von normalen Zellen vor der eradikativen Wirkung der chemotherapeutischen Verbindung bei einer Behandlung einer Tumorform mit einem inaktivierten p53-Stoffwechselweg.

15. Verwendung nach Anspruch 14, wobei die kombinierte Behandlung mit der schützenden Verbindung und der chemotherapeutischen Verbindung auf eine Vorbehandlung mit der schützenden Verbindung allein folgt.

16. Verwendung nach Anspruch 15, wobei die Vorbehandlung während eines Zeitraums erfolgt, der größer oder gleich der Dauer des Zellzyklus der proliferierenden normalen Zelle ist.

17. Verwendung nach einem der Ansprüche 14 bis 16, wobei die TumorForm eine Tumorform mit einem niedrigen Proliferationspotenzial ist.

18. Verwendung nach einem der Ansprüche 14 bis 17, wobei die Tumorform eine hyperproliferative Läsion ist, die durch ein Papillomavirus verursacht wird.

19. Verwendung einer schützenden Verbindung, wie sie in Anspruch 14 definiert ist, für die Herstellung eines Medikaments zum Schützen von normalen Zellen vor der eradikativen Wirkung einer chemotherapeutischen Verbindung bei einer Behandlung einer pathologischen Infektion, die durch Mikroorganismen verursacht wird, die keine p53-Funktion aufweisen.

20. Verwendung einer schützenden Verbindung, wie sie in Anspruch 14 definiert ist, für die Herstellung eines Medikaments zum Verhindern und Behandeln von Alopezie, die mit einer systemischen Verabreichung einer chemotherapeutischen Verbindung bei einer Behandlung von Zellen mit einem inaktivierten p53-Stoffwechselweg assoziiert ist.

21. Verwendung nach einem der Ansprüche 14 bis 20, wobei die schützende Verbindung ausgewählt ist aus der Gruppe bestehend aus Cytochalasinen, Jasplakinoliden, Chondramiden, Isoindolinonen und Latrunculinen, wobei Cytochalasin B ausgeschlossen ist.

22. Verwendung nach Anspruch 21, wobei die schützende Verbindung ausgewählt ist aus der Gruppe bestehend aus dem Cytochalasin D, Dihydrocytochalasin B, Jasplakinolid, Chondramid B und Latrunculin B.

23. Verwendung nach einem der Ansprüche 14 bis 22, wobei die chemotherapeutische Verbindung ausgewählt ist aus der Gruppe bestehend aus Folat-Inhibitoren, Nukleosid-Analoga, Nukleotidsynthese-Inhibitoren, Vinca-Alkaloiden, Taxanen, Colchicin-Derivaten, Podophyllotoxin-Derivaten und Topoisomerase-Inhibitoren.

24. Verwendung nach Anspruch 23, wobei die chemotherapeutische Verbindung ausgewählt ist aus der Gruppe bestehend aus Trifluorthymidin, Cytarabin, 6-Thioguanin, 6-Mercaptopurin, Gemcytabin, Fludarabin, Floxuridin, Ftorafur, Methotrexat, Trimetrexat, Raltitrexed, Edatrexat, Lometrexol, Hydroxyharnstoff, Vincristin, Vinblastin, Vinorelbin, Vindesin, Paclitaxel, Docetaxel, Irinotecan, Topotecan, 9-Amino-S(20)-camptothecin.

25. Pharmazeutische Zusammensetzung zum selektiven Eradizieren von Zellen mit einem inaktiven p53-Stoffwechselweg, umfassend therapeutisch wirksame Mengen einer schützenden Verbindung, einer chemotherapeutischen Verbindung und eines pharmazeutisch annehmbaren Vehikels, Trägers oder Hilfsmittels, wobei die chemotherapeutische Verbindung die Fähigkeit hat
- eine cytotoxische Wirkung gegen aktiv proliferierende Zellen auszuüben,
- das Überleben und proliferative Potenzial von Interphasezellen nicht zu beeinträchtigen,
wobei die schützende Verbindung die Fähigkeit hat
- die Zellteilung von normalen Zellen reversibel zu hemmen,
- die biologische Wirkung der chemotherapeutischen Verbindung nicht zu hemmen, wobei die Freisetzung der chemotherapeutischen Verbindung im Hinblick auf die Freisetzung der schützenden Verbindung verzögert ist.

26. Pharmazeutische Zusammensetzung nach Anspruch 25, wobei die chemotherapeutische Verbindung ausgewählt ist aus der Gruppe bestehend aus Folat-Inhibitoren, Nukleosid-Analoga, Nukleotidsynthese-Inhibitoren, Vinca-Alkaloiden, Taxanen, Colchicin-Derivaten, Podophyllotoxin-Derivaten und Topoisomeraseinhibitoren.

27. Pharmazeutische Zusammensetzung nach Anspruch 26, wobei die chemotherapeutische Verbindung ausgewählt ist aus der Gruppe bestehend aus Trifluorthymidin, Cytarabin, 6-Thioguanin, 6-Mercaptopurin, Gemcytabin, Fludarabin, Floxuridin, Ftorafur, Methotrexat, Trimetrexat, Raltitrexed, Edatrexat, Lometrexol, Hydroxyharnstoff, Vincristin, Vinblastin, Vinorelbin, Vindesin, Paclitaxel, Docetaxel, Irinotecan, Topotecan, 9-Amino-S(20)-camptothecin.

28. Pharmazeutische Zusammensetzung nach Anspruch 25, wobei die schützende Verbindung ausgewählt ist aus der Gruppe bestehend aus Cytochalasinen, Jasplakinoliden, Chondramiden, Isoindolinonen und Latrunculinen, wobei Cytochalasin B ausgeschlossen ist.

29. Pharmazeutische Zusammensetzung nach Anspruch 28, wobei die schützende Verbindung ausgewählt ist aus der Gruppe bestehend aus dem Cytochalasin D, Dihydrocytochalasin B, Jasplakinolid, Chondramid B und Latrunculin B.

30. Teilekit zum selektiven Eradizieren von Zellen mit einem inaktiven p53-Stoffwechselweg und selektiven Schützen von proliferierenden normalen Zellen, umfassend eine schützende Verbindung und eine chemotherapeutische Verbindung,
wobei die chemotherapeutische Verbindung die Fähigkeit hat
- eine cytotoxische Wirkung gegen aktiv proliferierende Zellen auszuüben,
- das Überleben und proliferative Potenzial von Interphasezellen nicht zu beeinträchtigen,
wobei die schützende Verbindung die Fähigkeit hat
- die Zellteilung von normalen Zellen reversibel zu hemmen
- die biologische Wirkung der chemotherapeutischen Verbindung nicht zu hemmen, wobei der Kit für die aufeinanderfolgende Verabreichung der schützenden Verbindung allein zuerst und anschließend der Assoziation der schützenden und chemotherapeutischen Verbindungen in einer *in vivo-* und/oder *ex vivo*-Therapie einer Tumorform mit einem inaktiven p53-Stoffwechselweg bestimmt ist.

31. Teilekit nach Anspruch 30, wobei die schützende Verbindung ausgewählt ist aus der Gruppe bestehend aus Cytochalasinen, Jasplakinoliden, Chondramiden, Isoindolinonen und Latrunculinen, wobei Cytochalasin B ausgeschlossen ist.

32. Teilekit nach Anspruch 30, wobei die schützende Verbindung ausgewählt ist aus der Gruppe bestehend aus Cytochalasin D, Dihydrocytochalasin B, Jasplakinolid, Chondramid B und Latrunculin B.

33. Teilekit nach Anspruch 30, wobei die chemotherapeutische Verbindung ausgewählt ist aus der Gruppe bestehend aus Folat-Inhibitoren, Nukleosid-Analoga, Nukleotidsynthese-Inhibitoren, Vinca-Alkaloiden, Taxanen, Colchicin-Derivaten, Podophyllotoxin-Derivaten und Topoisomerase-Inhibitoren.

34. Teilekit nach Anspruch 33, wobei die chemotherapeutische Verbindung ausgewählt ist aus der Gruppe bestehend aus Trifluorthymidin, Cytarabin, 6-Thioguanin, 6-Mercaptopurin, Gemcytabin, Fludarabin, Floxuridin, Ftorafur, Methotrexat, Trimetrexat, Raltitrexed, Edatrexat, Lometrexol, Hydroxyharnstoff, Vincristin, Vinblastin, Vinorelbin, Vindesin, Paclitaxel, Docetaxel, Irinotecan, Topotecan, 9-Amino-S(20)-camptothecin.

35. Teilekit nach einem der Ansprüche 30 bis 34, wobei die Tumorform eine hyperproliferative Läsion ist, die durch eine Papillomavirusinfektion verursacht wird.

36. Teilekit nach einem der Ansprüche 30 bis 34 in der Therapie einer pathologischen Infektion, die mit einem Mikroorganismus assoziiert ist, der keine p53-Funktion aufweist, wobei die Verabreichung der kombinierten Verbindungen auf die Verabreichung der schützenden Verbindung allein folgt.

37. Teilekit nach einem der Ansprüche 30 bis 34 zum selektiven Eradizieren von Zellen mit einer inaktiven p53-Funktion und zum selektiven Schützen von proliferierenden normalen Zellen in dem Verfahren nach einem der Ansprüche 1 bis 12.

## Revendications

1. Procédé pour protéger des cellules normales en prolifération dans une culture *in vitro* de l'action éradicatrice d'un composé chimiothérapique (composé de classe B), la culture comprenant lesdites cellules normales en prolifération et des cellules tumorales présentant une voie inactive de la p53,
ledit procédé comprenant l'administration du composé chimiothérapique en combinaison avec un composé protecteur (composé de classe A)à ladite culture,
le composé chimiothérapique ayant la capacité
- d'exercer une action cytotoxique envers les cellules proliférant activement,
- de ne pas affecter et le potentiel de survie et de prolifération des cellules en interphase, le composé protecteur ayant la capacité
- d'inhiber la cytodiérèse des cellules normales de façon réversible,
- de ne pas inhiber l'action biologique dudit composé chimiothérapique et,
dans lequel on applique un prétraitement au moyen dudit composé protecteur avant le traitement combiné au moyen de composés de classe A et de classe B et l'administration dudit composé protecteur donnent lieu à la protection d'au moins une partie desdites cellules normales en prolifération.

2. Procédé selon la revendication 1, dans lequel le prétraitement au moyen du composé protecteur donne lieu à l'arrêt en interphase d'au moins une partie desdites cellules normales.

3. Procédé selon la revendication 2, dans lequel le prétraitement est appliqué pendant un espace de temps supérieur ou égal à la durée du cycle cellulaire de ladite cellule normale en prolifération.

4. Procédé selon la revendication 1 ou 3, dans lequel, après le traitement combiné, on applique un post-traitement comprenant l'interruption de l'administration dudit composé de classe B et l'élimination dudit composé de classe B de la culture par lavage tout en maintenant l'administration dudit composé de classe A.

5. Procédé selon l'une quelconque des revendications 1 à 4, dans lequel le traitement combiné est appliqué pendant un espace de temps supérieur ou égal à la durée du cycle cellulaire desdites cellules tumorales présentant une voie inactivée de la p53.

6. Procédé selon la revendications 4 ou 5, dans lequel ladite étape de lavage est effectuée pendant un espace de temps supérieur ou égal à 3 heures.

7. Procédé selon l'une quelconque des revendications 1 à 4 dans lequel ledit traitement combiné, prétraitement et/ou post-traitement est répété deux fois ou davantage.

8. Procédé selon l'une quelconque des revendications 1 à 7, dans lequel ledit composé protecteur est choisi dans le groupe constitué par des cytochalasines, des jasplakinolides, des chondramides, des isoindolinones, et des latrunculines, à l'exclusion de la cytochalasine B.

9. Procédé selon la revendication 8, dans lequel ledit composé protecteur est choisi dans le groupe constitué par la cytochalasine D, la dihydrocytochalasine B, la jasplakinolide, la chondramide B et la latrunculine B.

10. Procédé selon l'une quelconque des revendications 1 à 9, dans lequel le composé chimiothérapique est choisi dans le groupe constitué par des inhibiteurs de folates, des analogues de nucléosides, des inhibiteurs de synthèse de nucléotides, des alcaloïdes de Vinca, des taxanes, des dérivés de la colchicine, des dérivés de la podophyllotoxine, et des inhibiteurs de topoisomérases.

11. Procédé selon la revendication 10, dans lequel le composé chimiothérapique est choisi dans le groupe constitué par la trifluorothymidine, la cytarabine, la 6-thioguanine, la 6-mercaptopurine, la gemcitabine, la fludarabine, la floxuridine, le ftorafur, le méthotrexate, le trimétrexate, le raltitrexed, l'édatrexate, le lométrexol, l'hydroxyurée, la vincristine, la vinblastine, la vinorelbine, la vindésine, le paclitaxel, le docétaxel, l'irinotécan, le topotécan, la 9-amino-S(20)-camptothécine.

12. Procédé selon l'une quelconque des revendications 1 à 11, dans lequel on emploie des cellules C3H10T1/2 ou des cellules dérivées de celles-ci comme cellules modèles.

13. Procédé selon l'une quelconque des revendications 1 à 2, dans lequel on emploie des cellules C3H10T1/2 ou des cellules dérivées de celle-ci comme cellules modèles pour identifier un composé protecteur et/ou un composé chimiothérapique.

14. Utilisation d'un composé protecteur ayant la capacité
- d'inhiber la cytodiérèse de cellules normales de façon réversible,
- de ne pas inhiber l'action biologique d'un composé chimiothérapique, lequel composé chimiothérapique a la capacité
- d'exercer une action cytotoxique envers des cellules proliférant activement, et
- de ne pas affecter le potentiel de survie et de prolifération de cellules en interphase,
pour la préparation d'un médicament pour protéger des cellules normales de l'action éradicatrice dudit composé chimiothérapique dans un traitement d'une forme tumorale présentant une voie inactivée de la p53.

15. Utilisation selon la revendication 14, dans laquelle le traitement combiné au moyen du composé protecteur et du composé chimiothérapique fait suite à un prétraitement à l'aide du composé protecteur seul.

16. Utilisation selon la revendication 15, dans laquelle le prétraitement est appliqué pendant un espace de temps supérieur ou égal à la durée du cycle cellulaire de ladite cellule normale en prolifération.

17. Utilisation selon l'une quelconque des revendications 14 à 16, dans laquelle ladite forme tumorale est une forme tumorale possédant un faible potentiel de prolifération.

18. Utilisation selon l'une quelconque des revendications 14 à 17, dans laquelle ladite forme tumorale est une lésion hyperproliférative induite par le papillomavirus.

19. Utilisation d'un composé protecteur tel que défini dans la revendication 14 pour la préparation d'un médicament protégeant des cellules normales de l'action éradicatrice d'un composé chimiothérapique dans un traitement d'une infection pathologique provoquée par des microorganismes ne présentant pas de fonction de la p53.

20. Utilisation d'un composé protecteur tel que défini dans la revendication 14 pour la préparation d'un médicament pour prévenir et traiter l'alopécie associée à une administration systémique d'un composé chimiothérapique dans un traitement de cellules présentant une voie inactivée de la p53.

21. Utilisation selon l'une quelconque des revendications 14 à 20 dans laquelle le composé protecteur est choisi dans le groupe constitué par des cytochalasines, des jasplakinolides, des chondramides, des isoindolinones, et des latrunculines, à l'exclusion de la cytochalasine B.

22. Utilisation selon la revendication 21 dans laquelle ledit composé protecteur est choisi dans le groupe constitué par la cytochalasine D, la dihydrocytochalasine B, la jasplakinolide, la chondramide B et la latrunculine B.

23. Utilisation selon l'une quelconque des revendications 14 à 22, dans laquelle le composé chimiothérapique est choisi dans le groupe constitué par des inhibiteurs de folates, des analogues de nucléosides, des inhibiteurs de synthèse de nucléotides, des alcaloïdes de Vinca, des taxanes, des dérivés de la colchicine, des dérivés de la podophyllotoxine, et des inhibiteurs de topoisomérases.

24. Utilisation selon la revendication 23, dans laquelle le composé chimiothérapique est choisi dans le groupe constitué par la trifluorothymidine, la cytarabine, la 6-thioguanine, la 6-mercaptopurine, la gemcitabine, la fludarabine, la floxuridine, le ftorafur, le méthotrexate, le trimétrexate, le raltitrexed, l'édatrexate, le lométrexol, l'hydroxyurée, la vincristine, la vinblastine, la vinorelbine, la vindésine, le paclitaxel, le docétaxel, l'irinotécan, le topotécan, la 9-amino-S(20)-camptothécine.

25. Composition pharmaceutique pour éradiquer sélectivement des cellules présentant une voie inactive de la p53 comprenant des quantités thérapeutiquement efficaces d'un composé protecteur, d'un composé chimiothérapique et d'un véhicule pharmaceutiquement acceptable, un transporteur ou agent auxiliaire, le composé chimiothérapique ayant la capacité
- d'exercer une action cytotoxique envers les cellules proliférant activement,
- de ne pas affecter le potentiel de survie et de prolifération des cellules en interphase, le composé protecteur ayant la capacité
- d'inhiber la cytodiérèse des cellules normales de façon réversible,
- de ne pas inhiber l'action biologique dudit composé chimiothérapique, dans laquelle la libération du composé chimiothérapique est retardée par rapport à la libération du composé protecteur.

26. Composition pharmaceutique selon la revendication 25, dans laquelle le composé chimiothérapique est choisi dans le groupe constitué par des inhibiteurs de folates, des analogues de nucléosides, des inhibiteurs de synthèse de nucléotides, des alcaloides de vinca, des taxanes, des dérivés de la colchicine, des dérivés de la podophyllotoxine, et des inhibiteurs de topoisomérases.

27. Composition pharmaceutique selon la revendication 26, dans laquelle ledit composé chimiothérapique est choisi dans le groupe constitué par la trifluorothymidine, la cytarabine, la 6-thioguanine, la 6-mercaptopurine, la gemcitabine, la fludarabine, la floxuridine, le ftorafur, le méthotrexate, le trimétrexate, le raltitrexed, l'édatrexate, le lométrexol, l'hydroxyurée, la vincristine, la vinblastine, la vinorelbine, la vindésine, le paclitaxel, le docétaxel, l'irinotécan, le topotécan, la 9-amino-S(20)-camptothécine.

28. Composition pharmaceutique selon la revendication 25, dans laquelle ledit composé protecteur est choisi dans le groupe constitué par des cytochalasines, des jasplakinolides, des chondramides, des isoindolinones, et des latrunculines, à l'exclusion de la cytochalasine B.

29. Composition pharmaceutique selon la revendication 28 dans laquelle ledit composé protecteur est choisi dans le groupe constitué par la cytochalasine D, la dihydrocytochalasine B, la jasplakinolide, la chondramide B et la latrunculine B.

30. Kit d'éléments pour éradiquer sélectivement les cellules présentant une voie inactive de la p53 et protéger sélectivement les cellules normales en prolifération constitué par un composé protecteur et un composé chimiothérapique, le composé chimiothérapique ayant la capacité
- d'exercer une action cytotoxique envers les cellules proliférant activement,
- de ne pas affecter le potentiel de survie et de prolifération des cellules en interphase, le composé protecteur ayant la capacité
- d'inhiber la cytodiérèse des cellules normales de façon réversible,
- de ne pas inhiber l'action biologique dudit composé chimiothérapique, le kit servant à l'administration séquentielle, d'abord, du composé protecteur seul, et ensuite de l'association des composés protecteur et chimiothérapique dans une thérapie *in vivo* et/ou *ex vivo* d'une forme tumorale présentant une voie inactive de la p53.

31. Kit d'éléments selon la revendication 30, dans lequel ledit composé protecteur est choisi dans le groupe constitué par des cytochalasines, des jasplakinolides, des chondramides, des isoindolinones, et des latrunculines, à l'exclusion de la cytochalasine B.

32. Kit d'éléments selon la revendication 30, dans lequel ledit composé protecteur est choisi dans le groupe constitué par la cytochalasine D, la dihydrocytochalasine B, la jasplakinolide, la chondramide B et la latrunculine B.

33. Kit d'éléments selon la revendication 30, dans lequel ledit composé chimiothérapique est choisi dans le groupe constitué par des inhibiteurs de folates, des analogues de nucléosides, des inhibiteurs de synthèse de nucléotides, des alcaloides de Vinca, des taxanes, des dérivés de la colchicine, des dérivés de la podophyllotoxine, et des inhibiteurs de topoisomérases.

34. Kit d'éléments selon la revendication 33, dans lequel ledit composé chimiothérapique est choisi dans le groupe constitué par la trifluorothymidine, la cytarabine, la 6-thioguanine, la 6-mercaptopurine, la gemcitabine, la fludarabine, la floxuridine, le ftorafur, le méthotrexate, le trimétrexate, le raltitrexed, l'édatrexate, le lométrexol, l'hydroxyurée, la vincristine, la vinblastine, la vinorelbine, la vindésine, le paclitaxel, le docétaxel, l'irinotécan, le topotécan, la 9-amino-S(20)-camptothécine.

35. Kit d'éléments selon l'une quelconque des revendications 30 à 34 dans lequel ladite forme tumorale est une lésion hyperproliférative induite par une infection du papillomavirus.

36. Kit d'éléments selon l'une quelconque des revendications 30 à 34 dans la thérapie d'une infection pathologique associée à un microorganisme ne présentant pas de fonction de la p53, dans lequel l'administration des composées combinés suit l'administration du composé protecteur seul.

37. Kit d'éléments selon l'une quelconque des revendications 30 à 34 pour éradiquer sélectivement les cellules présentant une fonction inactive de la p53 et protéger sélectivement les cellules normales en prolifération dans le procédé selon l'une quelconque des revendications 1 à 12.
